# EUROPEAN PATENT APPLICATION

(11) **EP 1 517 144 A2**
(43) Date of publication of application: **23.03.2005**
(21) Application number: 04078216.1
(22) Date of filing: 30.11.1999
(51) Int. Cl.: G01N 33/53, C12N 15/12, C07K 14/705, C12N 5/10, C07K 16/28

(54) **Method of identifying an antibody binding to and modulating the activity of a human vanilloid receptor**

(30) Priority: 01.12.1998 GB 9826359
(62) Divisional of application: 99963344.9
(71) Applicant: GLAXO GROUP LIMITED, Greenford, Middlesex UB6 0NN (GB)
(72) Inventor: Delany, Natalie Samantha, Stevenage Hertfordshire SG1 2NY (GB); Sanseau, Philippe, Stevenage Hertfordshire SG1 2NY (GB); Tate, Simon Nicholas, Stevenage Hertfordshire SG1 2NY (GB)
(74) Representative: Nash, Guy Cameron

(57) **Abstract**

The invention provides novel human vanilloid receptor (hVR) proteins, in particular hVR1 and hVR3, nucleotide sequences encoding for the novel hVR proteins, and hVR proteins for use in a method for screening for agents useful in the treatment or prophylaxis of disorders which are responsive to modulation of hVR activity in a human patient. The invention also provides expression vectors comprising said nucleotide sequences, stable cell lines comprising said expression vectors, antibodies specific for the novel hVR proteins, methods for the identification of compounds which exhibit hVR modulating activity, compounds identifiable and identified by such methods, and methods of treatment or prophylaxis of disorders which are responsive to modulation of hVR activity in a human patient.

## Description

### Field of the Invention

The present invention relates to human vanilloid receptor (hVR) proteins and to related nucleotide sequences, expression vectors, cell lines, antibodies screening methods, compounds, methods of production and methods of treatment, as well as other related aspects.

### Background of the Invention

Capsaicin, the irritant in hot peppers and a member of the vanilloid family activates a sub-group of sensory neurons: the nociceptors. These neurons transmit nociceptive and thermoceptive pain information back to pain-processing centres in the central nervous system such as the spinal cord and the brain. They are also sites for the release of pro-inflammatory mediators in the periphery (1). Nociceptors show heterogeneity in their sensitivity to capsaicin. Excitation and prolonged exposure of these neurons to capsaicin is followed by a refractory state known as desensitisation (2) when they become insensitive to capsaicin and other noxious stimuli (3). The long-term response to insensitivity could be explained by death of the nociceptors or destruction of its peripheral terminals (4). Because of the desensitisation phenomenon, capsaicin has been used therapeutically for decades as an analgesic agent for the treatment of pain in a range of disorders (5).

It has been speculated that the endogenous target for capsaicin plays an important function in the detection of painful stimuli. It has been shown by electrophysiological and biochemical studies that capsaicin induces a flux of cations in dorsal root ganglion (DRG) neurons (6,7). Because other vanilloid derivatives show responses in a dose dependent manner (8,9) a receptor is the most likely candidate to explain the mechanism. Therefore, based on indirect evidence it has been anticipated that these actions of capsaicin (excitation / desensitisation) are mediated by a specific membrane-bound receptor named vanilloid receptor (10).

Evidence for the existence of a vanilloid receptor came from binding experiments with resiniferatoxin (RTX), a capsaicin analog (11), and a competitive antagonist of capsaicin, capsazepine (12). Vanilloid receptors have been visualised by using ([³H]-RTX) autoradiography in dorsal root ganglia (DRG) and spinal cord of different species including man (13,14).

Recently, a rat vanilloid receptor termed VR1 has been identified using an expression-cloning strategy to isolate the complementary DNA (cDNA) encoding the corresponding protein from a rat DRG cDNA library (15). The cDNA clone was completely sequenced. The rat VR1 cDNA has an open reading frame of 2,514 nucleotides and encodes for a protein of 838 amino acids with a predicted relative molecular mass of 95,000. Analysis of the amino acid sequence identified 6 potential transmembrane regions with a short hydrophobic stretch between the transmembrane regions 5 and 6. The N-terminus (amino terminal) contains three ankyrin repeat domains. No motifs have been identified at the C-terminus (carboxy terminal).

It has been noted that rat VR1 transfected cells exhibit an increase in calcium levels after heat treatment and it has been suggested that *in vivo* VR1 and vanilloid receptors are involved in detection of noxious heat (but not innocuous heat). It has also been proposed that protons could act as modulators of the vanilloid receptors (16, 17, 18).

While it has been recognised that the rat capsaicin receptor, VR1, is a member of the family of non-selective ion channels that are gated by ligands and that it is involved in pain sensation, the natural ligand of VR1 remains unknown. It is therefore suggested that human vanilloid receptor sub-types may provide targets for the development of novel analgesic agents (agonists and antagonists) and agents which may interact with other disorders.

Accordingly, it is an object of the present invention to locate and characterise human vanilloid receptors. Other objects of the present invention will become apparent from the following detailed description thereof.

### Summary of the Invention

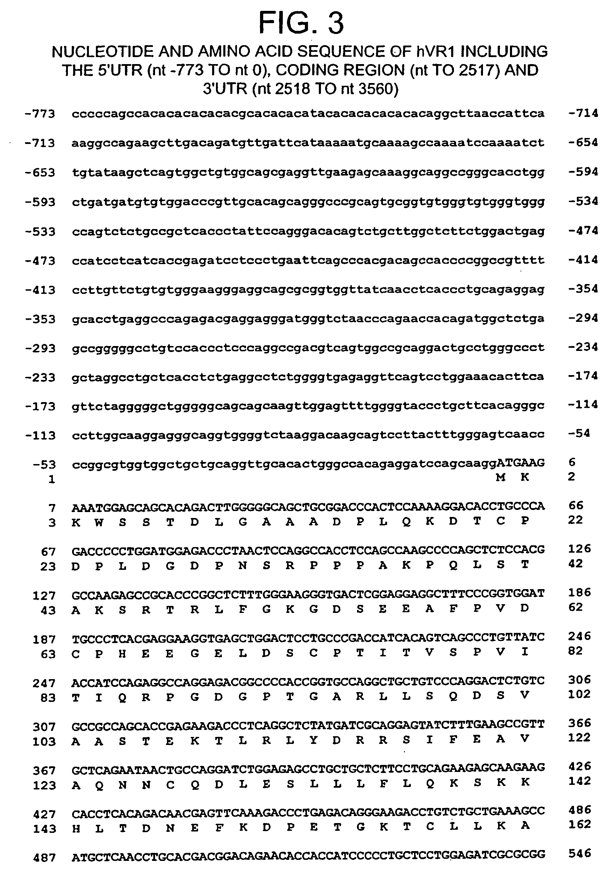

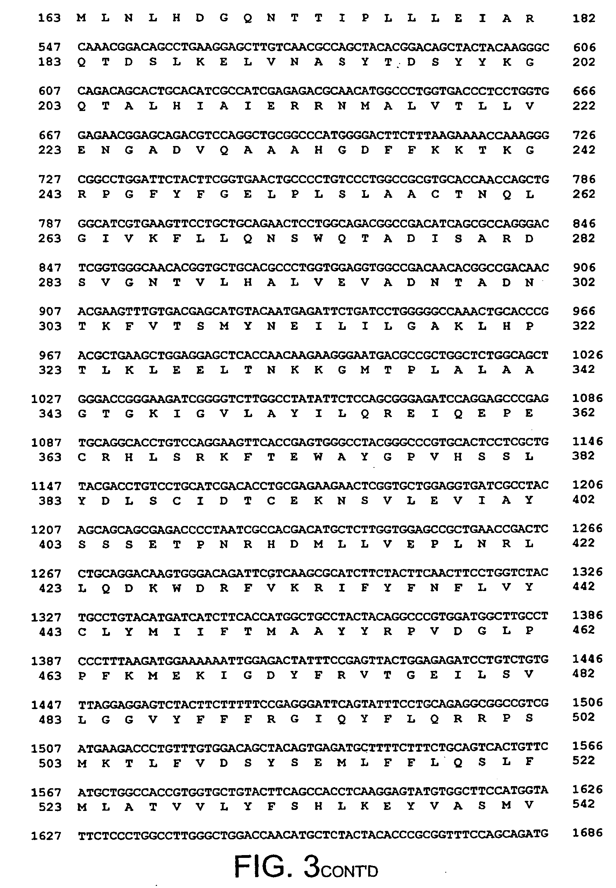

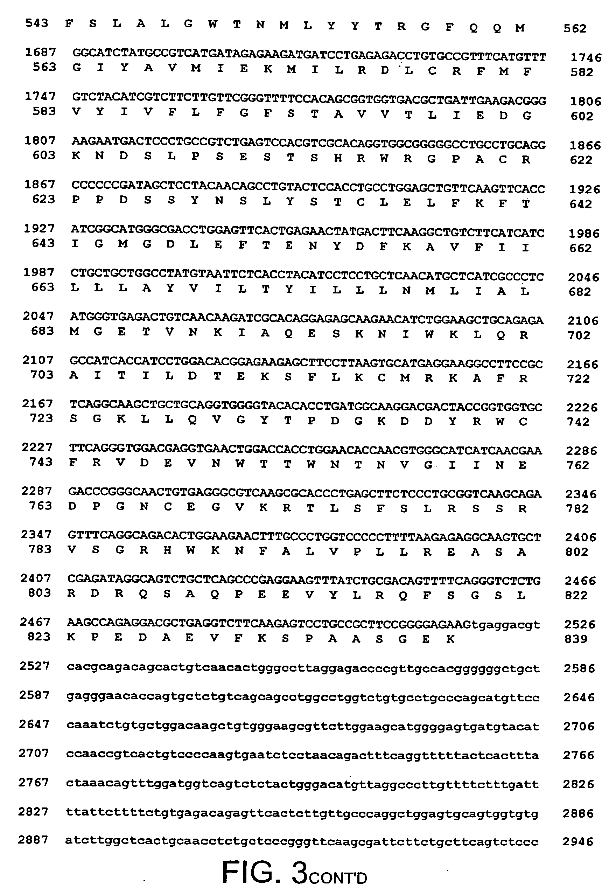

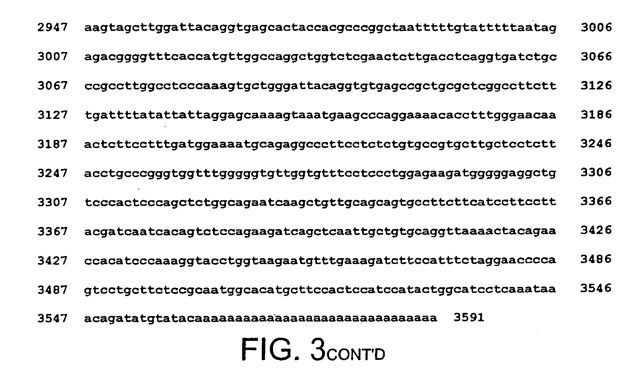

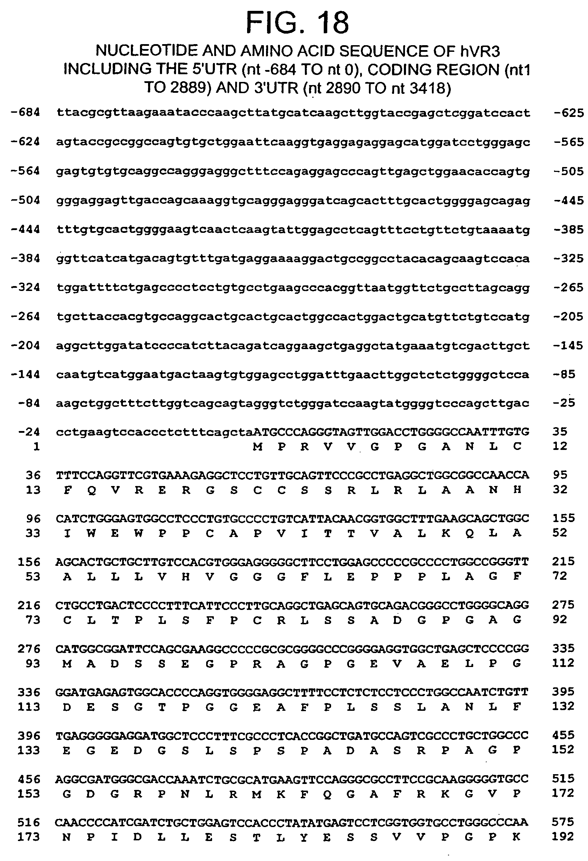

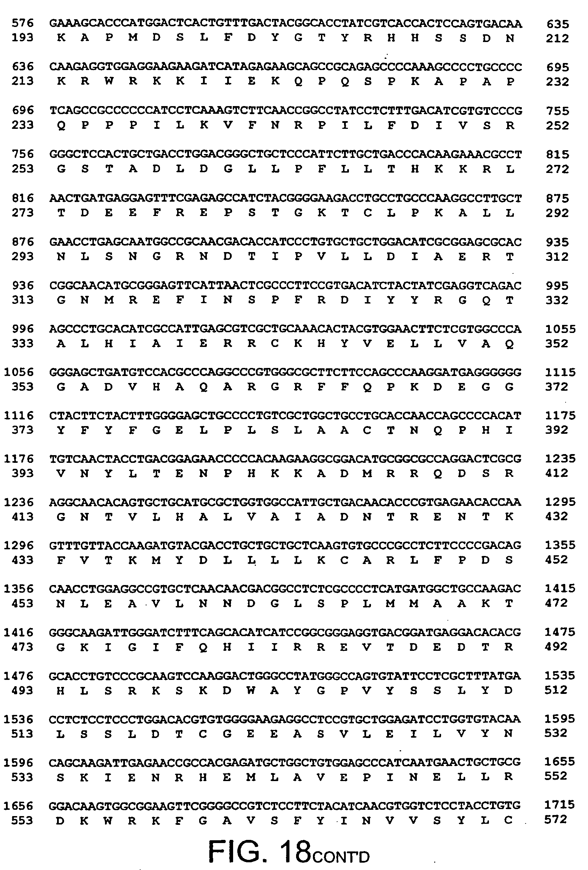

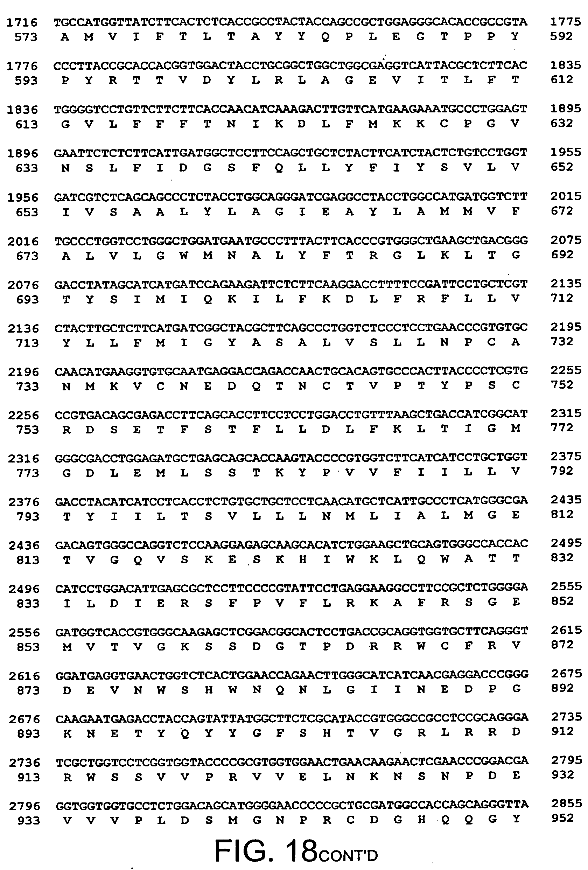

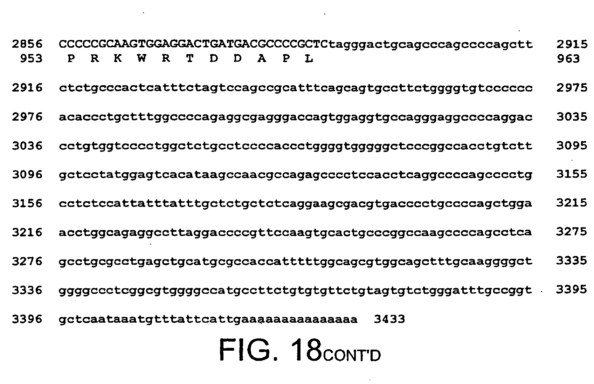

According to one embodiment of the present invention there is provided an isolated human vanilloid receptor (hVR) protein or a variant thereof. Preferably the hVR protein is an hVR1 or hVR3 protein or a variant thereof. In a particularly preferred aspect of the invention the hVR protein has an amino acid sequence as shown in figure 3 or in figure 18.

According to another aspect of the invention, there is provided a human vanilliod receptor (hVR) protein or a variant thereof, preferably hVR1 or hVR3 or a variant thereof, for use in a method of screening for agents useful in the treatment or prophylaxis of a disorder which is responsive to the modulation of hVR activity, preferably hVR1 or hVR3 activity, in a human patient. Preferably the disorder is pain, neuropathic pain, inflammatory pain, chronic pain, post-operative pain, rheumatoid arthritic pain, neuropathies, neuralgia, algesia, neurodegeneration, nerve injury, stroke, ischaemia migraine, irritable bowl syndrome (IBS), a respiratory disorder such as asthma or chronic obstructive pulmonary disease (COPD), a urological disorder such as diabetic neuropathy, incontinence and interstitial cystitis, or an inflammatory disorder.

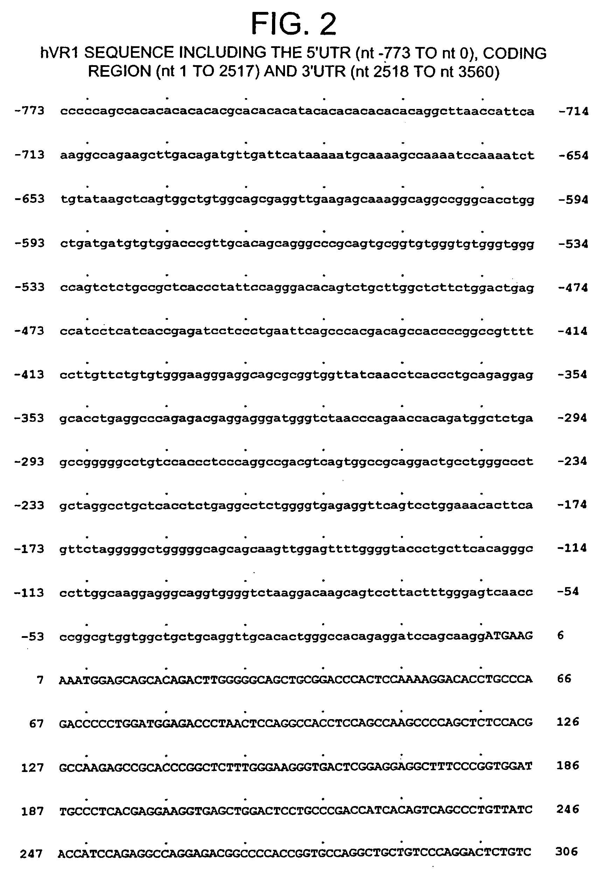

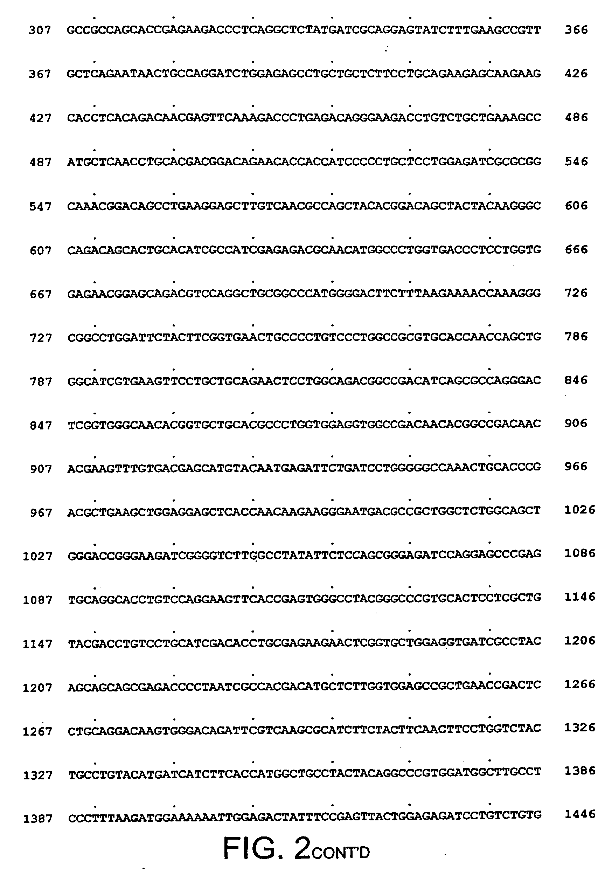

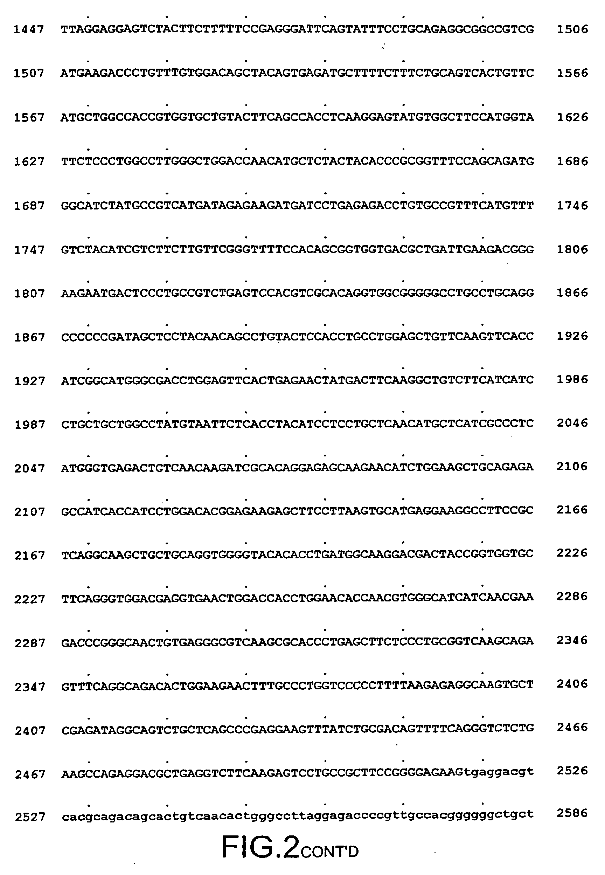

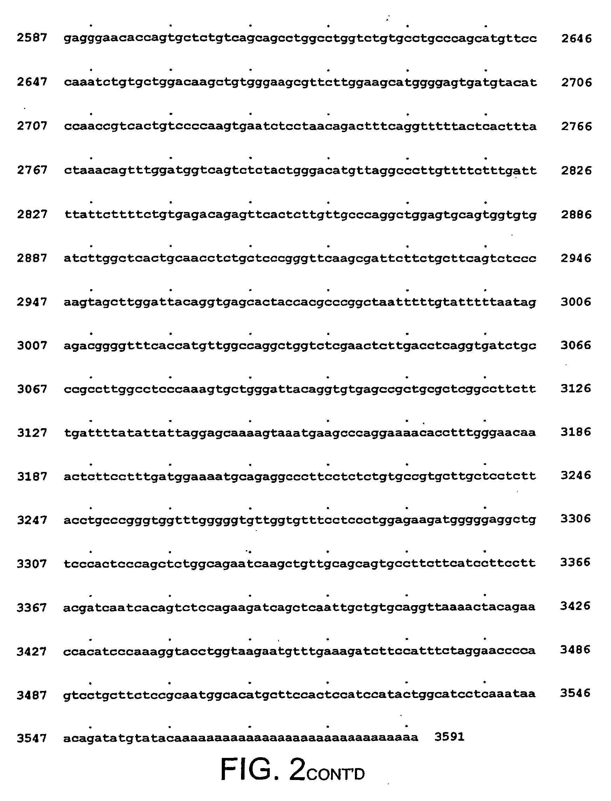

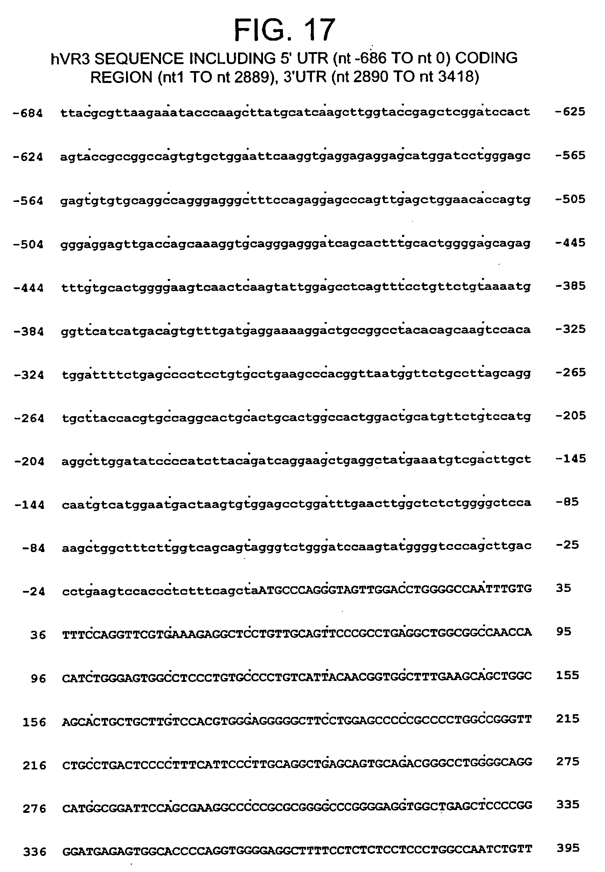

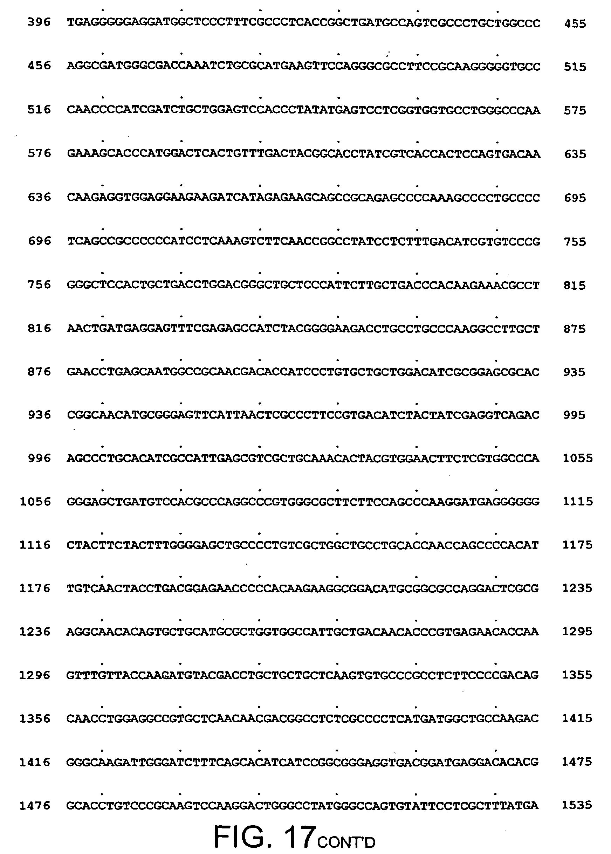

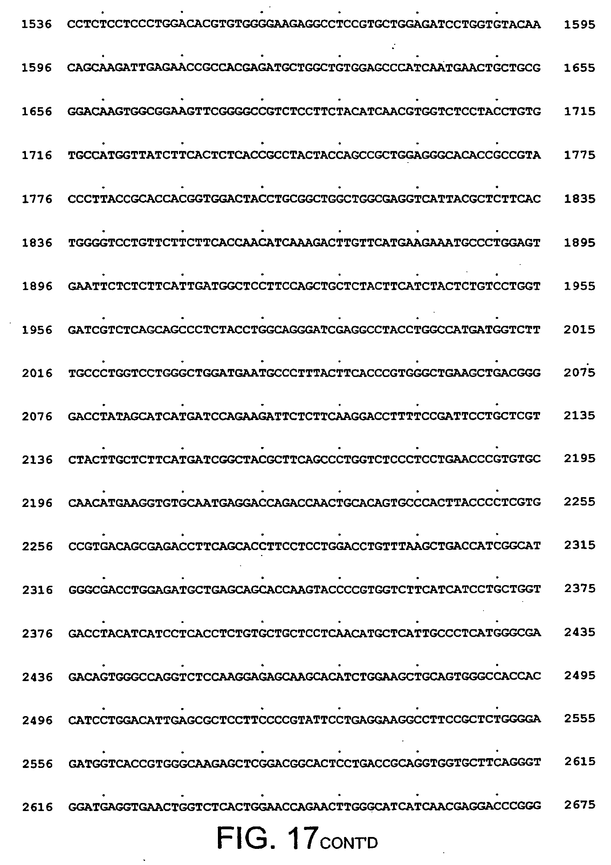

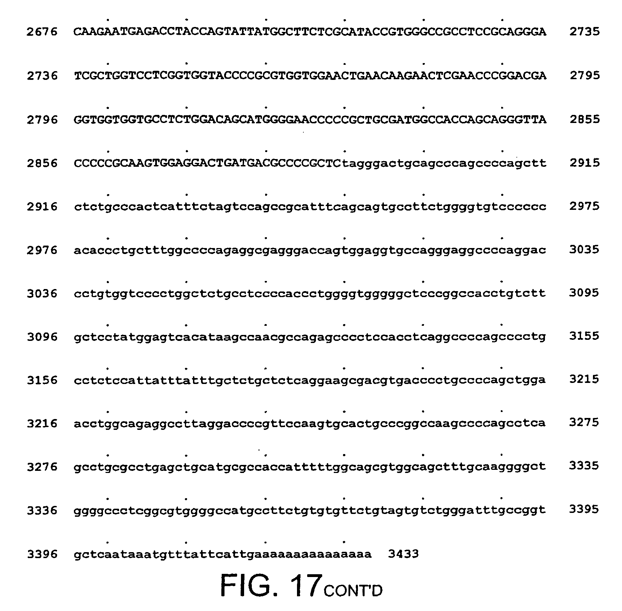

According to another aspect of the invention there is provided a nucleotide sequence encoding a human vanilloid receptor (hVR) protein or a variant thereof as hereinbefore described, or a nucleotide sequence that is complementary thereto. Preferably the nucleotide sequence encodes an hVR1, hVR3 protein or variant thereof or a nucleotide sequence which is complementary thereto. Particularly preferably the nucleotide sequence is as shown in figure 2 and figure 17.

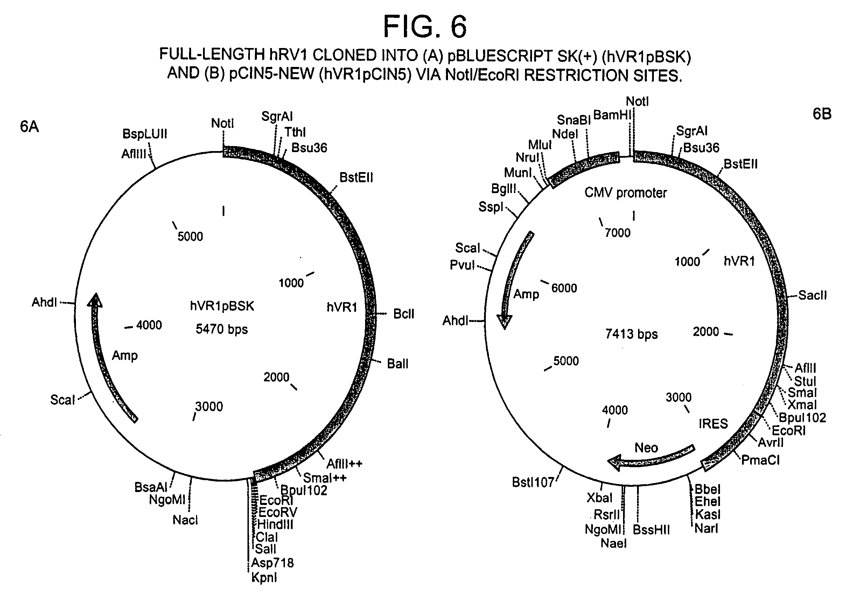

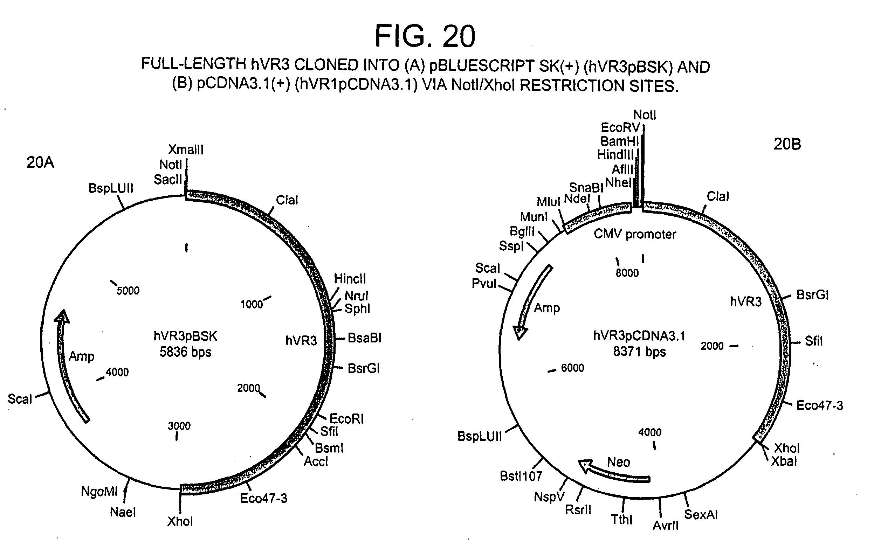

According to another aspect of the invention there is provided an expression vector comprising a nucleic acid sequence as referred to above which is capable of expressing an hVR protein as hereinbefore described or a variant thereof, preferably hVR1 or hVR3 or a variant thereof. Preferably the expression vector is as displayed in figure 6 or figure 20.

According to another aspect of the invention there is provided a stable cell line comprising an expression vector as referred to above which is capable of expressing an hVR protein as hereinbefore described or a variant thereof, preferably hVR1 or hVR3 or a variant thereof. The stable cell line is preferably a modified mammalian cell line, preferably HEK293, CHO, COS, HeLa or BHK although transient expression may be preferred in *Xenopus* oocytes.

According to another aspect of the invention there is provided an antibody specific for an hVR protein as hereinbefore described or a variant thereof, preferably specific for hVR1 or hVR3 or a variant thereof.

According to another aspect of the invention there is provided a method for identification of a compound which exhibits hVR modulating activity, comprising contacting an hVR protein as hereinbefore described or a variant thereof, preferably hVR1 or hVR3 or a variant thereof, with a test compound and detecting modulating activity or inactivity.

According to another aspect of the invention there is provided a compound which modulates hVR activity, preferably that of hVR1 or hVR3, identifiable by the method referred to above.

According to another aspect of the invention there is provided a compound which modulates hVR activity, preferably that of hVR1 or hVR3, identifiable by the method referred to above, for use in therapy.

According to another aspect of the invention there is provided the use of a compound which modulates hVR activity, preferably that of hVR1 or hVR3, identifiable by the method referred to above, in the manufacture of a medicament for treatment or prophylaxis of a disorder which is responsive to the modulation of hVR activity, preferably hVR1 activity or hVR3 activity, in a human patient. Preferably the disorder is pain, neuropathic pain, inflammatory pain, chronic pain, post-operative pain, rheumatoid arthritic pain, neuropathies, neuralgia, algesia, neurodegeneration, nerve injury, stroke, ischaemia migraine, irritable bowl syndrome (IBS), a respiratory disorder such as asthma or chronic obstructive pulmonary disease (COPD), a urological disorder such as neuropathy, incontinence or interstitial cystitis, or an inflammatory disorder.

According to another aspect of the invention there is provided a method of treatment or prophylaxis of a disorder which is responsive to modulation of hVR, preferably hVR1 or hVR3, activity in a human patient which comprises administering to said patient an effective amount of a compound identifiable by the method referred to above. Preferably the disorder is pain, neuropathic pain, inflammatory pain, chronic pain, post-operative pain, rheumatoid arthritic pain, neuropathies, neuralgia, algesia, neurodegeneration, nerve injury, stroke, ischaemia migraine, irritable bowl syndrome (IBS), respiratory disorders such as asthma and chronic obstructive pulmonary disease (COPD) and urological disorders including diabetic neuropathy, incontinence and interstitial cystitis and inflammatory disorders.

According to another aspect of the invention there is provided a compound which modulates hVR activity, preferably that of hVR1 or hVR3, identifiable by the method referred to above, excluding the compounds capsaicin, resiniferatoxin, piperine, zingerone, polydodial, warburganal, aframodial, cinnamodial, cinnamosmolide, cinnamolide, isovelleral, scalaradial, ancistrodial, β-acaridia), scutigeral, merulidial, anandamide and capsazepine.

According to another aspect of the invention there is provided a compound which modulates hVR activity, preferably that of hVR1 or hVR3, identifiable by the method referred to above, excluding the compounds capsaicin, resiniferatoxin, piperine, zingerone, polydodial, warburganal, aframodial, cinnamodial, cinnamosmolide, cinnamolide, isovelleral, scalaradial, ancistrodial, β-acaridial, scutigeral, merulidial, anandamide and capsazepine, for use in therapy.

According to another aspect of the invention there is provided the use of a compound which modulates hVR activity, preferably that of hVR1 or hVR3, identifiable by the method referred to above, excluding the compounds capsaicin, resiniferatoxin, piperine, zingerone, polydodial, warburganal, aframodial, cinnamodial, cinnamosmolide, cinnamolide, isovelleral, scalaradial, ancistrodial, β-acaridial, scutigeral, merulidial, anandamide and capsazepine, in the manufacture of a medicament for treatment or prophylaxis of a disorder which is responsive to the modulation of hVR activity, preferably hVR1 activity or hVR3 activity, in a human patient. Preferably the disorder is pain, neuropathic pain, inflammatory pain, chronic pain, post-operative pain, rheumatoid arthritic pain, neuropathies, neuralgia, algesia, neurodegeneration, nerve injury, stroke, ischaemia migraine, irritable bowl syndrome (IBS), a respiratory disorder such as asthma or chronic obstructive pulmonary disease (COPD), a urological disorder such as neuropathy, incontinence or interstitial cystitis, or an inflammatory disorder.

According to another aspect of the invention there is provided a method of treatment or prophylaxis of a disorder which is responsive to modulation of hVR, preferably hVR1 or hVR3, activity in a human patient which comprises administering to said patient an effective amount of a compound identifiable by the method referred to above, excluding the compounds capsaicin, resiniferatoxin, piperine, zingerone, polydodial, warburganal, aframodial, cinnamodial, cinnamosmolide, cinnamolide, isovelleral, scalaradial, ancistrodial, β-acaridial, scutigeral, merulidial, anandamide and capsazepine. Preferably the disorder is pain, neuropathic pain, inflammatory pain, chronic pain, post-operative pain, rheumatoid arthritic pain, neuropathies, neuralgia, algesia, neurodegeneration, nerve injury, stroke, ischaemia migraine, irritable bowl syndrome (IBS), respiratory disorders such as asthma and chronic obstructive pulmonary disease (COPD) and urological disorders including diabetic neuropathy, incontinence and interstitial cystitis and inflammatory disorders.

According to another aspect of the invention there is provided a compound identified by the method referred to above.

According to another aspect of the invention there is provided a compound identified by the method referred to above, for use in therapy.

According to another aspect of the invention there is provided the use of a compound identified by the method referred to above in the manufacture of a medicament for treatment or prophylaxis of a disorder which is responsive to the modulation of hVR activity, preferably hVR1 activity or hVR3 activity, in a human patient. Preferably the disorder is pain, neuropathic pain, inflammatory pain, chronic pain, post-operative pain, rheumatoid arthritic pain, neuropathies, neuralgia, algesia, neurodegeneration, nerve injury, stroke, ischaemia migraine, irritable bowl syndrome (IBS), a respiratory disorder such as asthma or chronic obstructive pulmonary disease (COPD), a urological disorder such as neuropathy, incontinence or interstitial cystitis, or an inflammatory disorder.

According to another aspect of the invention there is provided a method of treatment or prophylaxis of a disorder which is responsive to modulation of hVR, preferably hVR1 or hVR3, activity in a human patient which comprises administering to said patient an effective amount of a compound identified by the method referred to above. Preferably the disorder is pain, neuropathic pain, inflammatory pain, chronic pain, post-operative pain, rheumatoid arthritic pain, neuropathies, neuralgia, algesia, neurodegeneration, nerve injury, stroke, ischaemia migraine, irritable bowl syndrome (IBS), respiratory disorders such as asthma and chronic obstructive pulmonary disease (COPD) and urological disorders including diabetic neuropathy, incontinence and interstitial cystitis and inflammatory disorders.

According to another aspect of the invention there is provided a method of producing an hVR protein as hereinbefore described or a variant thereof, preferably hVR1 or hVR3 or a variant thereof, comprising introducing into an appropriate cell line a suitable vector comprising a nucleotide sequence encoding for an hVR protein or a variant thereof, preferably hVR1 or hVR3 or a variant thereof, under conditions suitable for obtaining expression of the hVR protein or a variant thereof, preferably hVR1 or hVR3 or a variant thereof.

### Brief Description of the figures

Figure 1 is an alignment of hVR1 *in silico* derived clusters with rat VR1.
Figure 2 displays the human VR1 nucleotide sequence including the 5'UTR (nt - 773 to nt 0), coding region (nt 1 to 2517) and 3'UTR (nt 2518 to nt 3560).
Figure 3 illustrates the nucleotide and encoded amino acid sequence of the human VR1sequence.
Figure 4 depicts the amino acid sequence of the hVR1 gene, the shading denotes predicted trans-membrane regions (boxed) and the ankyrin binding domains (unboxed). The predicted phosphorylation sites are underlined.
Figure 5 is a comparison of the amino acid sequences of the rat (rVR1) and human (hVR1) vanilloid receptors.
Figure 6 illustrates constructs pBluescriptSK(+) (A) and pCIN5-new (B) with the full length HVR1 gene cloned via Notl and EcoRI restriction sites.
Figure 7 shows a Slot Blot hybridisation with hVR1 probe with positive labelling of both rat and human DRG mRNA.
Figure 8 displays a Western blot probed with anti-VR1 antibodies with the arrow indicating the VR1 specific protein.
Figure 9 shows localisation of VR1 in rat DRG tissue sections, the arrow points to VR1 expressing small diameter (<25µn) neurone cell bodies.
Figure 10 depicts the *in situ* localisation of VR1 in human DRG sections (A) and human skin (B).
Figure 11 illustrates the functional response to capsaicin and blockade by capsazepine (CPZ) (A) with the current voltage relationship plotted in (B) on human VR-1 channels, transiently expressed in HEK293T cells.
Figure 12 shows capsaicin-induced desensitisation of human VR-1 channels in the presence of 2mM external calcium (A), maximum current (65mV) against time (B) and current voltage relationship in the absense of Ca²⁺ (C).
Figure 13 shows the influx of calcium into transiently transfected HEK293T cells over a time course in the presence of agonist capsaicin, anandamide and resiniferatoxin in the absence (A, B, D and F) or presence (C, E, G) of the antagonist, capsezipine.
Figure 14 illustrates a graphical presentation the results shown in figure 13 examining the response of hVR1 transfected HEK293T cells over time before and after exposure to agonists: capsaicin, anandamide and resiniferatoxin in the absence (A, B, D and F) or presence (C, E, G) of the antagonist, capsezipine.
Figure 15 displays the proposed assay strategy to carry out drug screening.
Figure 16 displays an alignment of *in silico* derived hVR3 specific clusters with rat VR1.
Figure 17 depicts the hVR3 nucleotide sequence including the 5' UTR (nt -686 to nt 0) Coding region (nt1 to nt 2889), 3'UTR (nt 2890 to nt 3418).
Figure 18 shows the nucleotide and amino acid sequence of hVR3.
Figure 19 is of the amino acid sequence of hVR3, the shading denotes predicted trans-membrane regions (boxed) and the ankyrin binding domains (unboxed).
Figure 20 displays constructs pBluescriptSK(+) (A) and pCDNA3.1 (+) (B) with the full length hVR3 gene cloned via Notl and Xhol restriction sites.
Figure 21 illustrates a multiple comparison of the amino acid sequences of the rat VR1 and the human vanilloid receptors: hVR1, hVRL-1 and hVR3.
Figure 22 Northern Blot hybridisation with hVR3 probe with strong signals detected in trachea (A), prostate (B), placenta, kidney and pancreas (C).

### Detailed Description of the Invention

Throughout the present specification and the accompanying claims the words "comprise" and "include" and variations such as "comprises", "comprising", "includes" and "including" are to be interpreted inclusively. That is, these words are intended to convey the possible inclusion of other elements or integers not specifically recited, where the context allows.

As referred to above, the present invention relates to isolated human vanilloid receptor (hVR) proteins, and in particular to the human vanilloid receptors which will be termed respectively human vanilloid receptors 1 and 3 (hVR1, and hVR3), sequence information for which is provided in figures 2 (HVR1) and 17 (hVR3). In the context of this invention the term "isolated" is intended to convey that the receptor protein is not in its native state, insofar as it has been purified at least to some extent or has been synthetically produced, for example by recombinant methods. The term "isolated" therefore includes the possibility of the receptor protein being in combination with other biological or non-biological material, such as cells, suspensions of cells or cell fragments, proteins, peptides, organic or inorganic solvents, or other materials where appropriate, but excludes the situation where the receptor protein is in a state as found in nature.

Routine methods, as further explained in the subsequent experimental section, can be employed to purify and/or synthesise the receptor proteins according to the invention. Such methods are well understood by persons skilled in the art, and include techniques such as those disclosed in Sambrook, J. et al. (28), the disclosure of which is included herein in its entirety by way of reference.

By the term "variant" what is meant throughout the specification and claims is that other peptides or proteins which retain the same essential character of the human vanilloid receptor proteins for which sequence information is provided, are also intended to be included within the scope of the invention. For example, other peptides or proteins with greater than about 80%, preferably at least 90% and particularly preferably at least 95% homology with the sequences provided are considered as variants of the receptor proteins. Such variants may include the deletion, modification or addition of single amino acids or groups of amino acids within the protein sequence, as long as the peptide maintains the biological functionality of a human vanilloid receptor. This biological functionality can of course be assessed by conducting binding studies with known vanilloid modulators such as capsaicin, capsazepine (12) and resiniferatoxin (11).

Human VR1 is preferentially expressed in human dorsal root ganglia (DRG) and relative to hVR3 has the highest sequence homology with the rat VR1. Therefore, hVR1 is likely to be the human orthologue to rat VR1. hVR3 is less similar to rat VR1 and is expressed in a wider range of tissues. Nucleotide sequence analysis of hVR1 reveals a 2517bp open reading frame which encodes an 839 amino acid protein (see figures 2, 3 and 4). This deduced hVR1 protein sequence is 86 % identical to the rat VR1 (15) and shares many of its characteristics such as 6 transmembrane regions with an hydrophobic stretch between transmembrane 5 and 6 and an N-terminus which contains 3 ankyrin repeat domains. Similarly hVR3 has an open reading frame of 2889bp open reading frame which encodes a 963 amino acid protein (see figures 17, 18 and 19). The deduced hVR3 protein is 46 % identical to rat VR1 and 44 % identical to hVR1 sharing many of VR1's characteristics such as 6 transmembrane regions with an hydrophobic stretch between transmembrane 5 and 6 and an N-terminus which contains 3 ankyrin repeat domains.

The invention also includes nucleotide sequences which encode for human vanilloid receptor proteins or variants thereof as well as nucleotide sequences which are complementary thereto. Preferably the nucleotide sequence is a DNA sequence and most preferably, a cDNA sequence. Preferably the proteins are hVR1, hVR3 or variants thereof. Such nucleotides can be isolated or synthesised according to methods well know in the art. See reference 28, the disclosure of which is included herein in its entirety by way of reference.

The present invention also includes expression vectors which comprise nucleotide sequences encoding for the hVR, preferably hVR1 or hVR3, receptor proteins or variants thereof. A further aspect of the invention relates to an expression vector comprising nucleotide sequences encoding for hVR1 or hVR3 receptor proteins or variants thereof. Such expression vectors are routinely constructed in the art of molecular biology and may for example involve the use of plasmid DNA and appropriate initiators, promoters, enhancers and other elements, such as for example polyadenylation signals which may be necessary, and which are positioned in the correct orientation, in order to allow for protein expression. Suitable vectors for use in practicing the present invention include pBluescript (Stratagene), pCR-Script (Stratagene), pCR2.1-TOPO (Invitrogen), pCRII-TOPO (Invitrogen), pCR-Blunt (Invitrogen), with vectors such as pCIN (32) (available from Clontech as pIRES-neo), pCDNA 3.1 (Invitrogen) or pClneo (Promega) required for mammalian expression. Appropriate methods can be effected by following protocols described in many standard laboratory manuals (28, 29).

The invention also includes cell lines which have been modified to express the novel receptor. Such cell lines include transient, or preferably stable higher eukaryotic cell lines, such as mammalian cells or insect cells, lower eukaryotic cells, such as yeast or prokaryotic cells such as bacterial cells. Particular examples of cells which have been modified by insertion of vectors encoding for the receptor proteins according to the invention include HEK293T cells and *Xenopus* oocytes. Preferably the cell line selected will be one which is not only stable, but also allows for mature glycosylation and cell surface expression of the inventive receptor. Representive examples of appropriate hosts include animal cells such as HEK293, CHO, COS, HeLa and BHK.

It is also possible for the receptors of the invention to be transiently expressed in a cell line or on a membrane, such as for example in a baculovirus expression system. Such systems, which are adapted to express the receptors according to the invention, are also included within the scope of the present invention.

In particular, the functional hVR protein may include hVR receptor proteins selected from hVR1 and hVR3 and thereof or even other hVR protein subtypes or splice variants which have not yet been identified.

According to another aspect, the present invention also relates to antibodies, preferably monoclonal antibodies, which have been raised by standard techniques and are specific for the receptor proteins or variants thereof according to the invention. Such antibodies could for example, be useful in purification, isolation or screening involving immuno precipitation techniques and may be used as tools to further ellucidate hVR, preferably hVR1 or hVR3, protein function, or indeed as therapeutic agents in their own right. Antibodies may also be raised against specific epitopes of the receptors according to the invention.

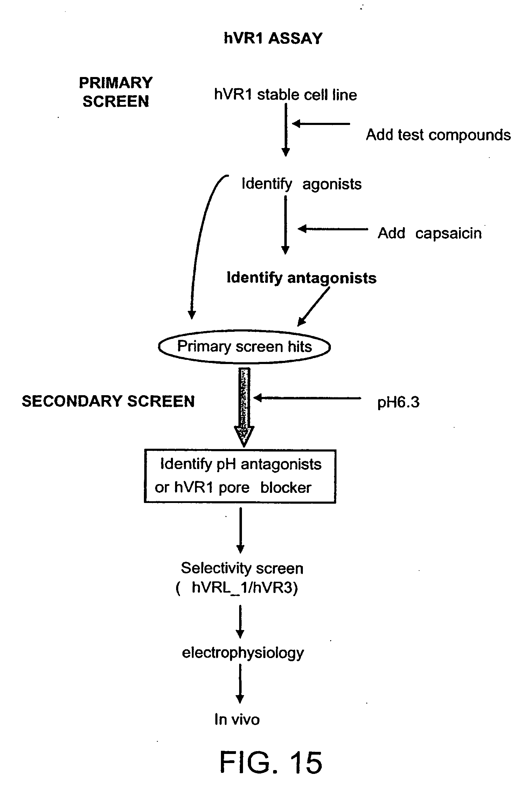

An important aspect of the present invention is the use of receptor proteins according to the invention in screening methods designed to identify compounds which act as receptor ligands and which may be useful to modulate receptor activity. In general terms, such screening methods will involve contacting the receptor protein concerned, preferably hVR1 or hVR3, with a test compound and then detecting modulation in the receptor activity, or indeed detecting receptor inactivity, which results. For further details on the screening strategy refer to figure 15. The present invention also includes within its scope those compounds which are identified as possessing useful hVR, preferably hVR1 or hVR3, modulation activity, by the screening methods referred to above. The screening methods comprehended by the invention are generally well known to persons skilled in the art. High throughput screens may include fluorescence based assays using the Fluorometric Imaging Plate Reader (FLIPR) with calcium sensitive dyes, and reporter gene assays using calcium sensitive photoproteins that emit light on the influx of calcium and can be detected using an Imaging system. Secondary screens may involve electrophysiological assays utilising patch clamp technology to identify small molecules, antibodies, peptides, proteins or other types of compounds that interact with hVR, preferably hVR1 or hVR3, to modulate activity. Tertiary screens may involve the study of modulators in well characterised rat and mouse models of pain. These models of pain include, but are not restricted to, intraplantar injection of inflammatory agents such as carageenan, formalin and complete freunds adjuvant (CFA). Models of neuropathic pain such as loose ligature of the sciatic nerve are also included. Other screens may involve the study of modulators in human volunteers subject to topically applied capsaicin.

Another aspect of the present invention is the use of compounds which have been identified by screening techniques referred to above in the treatment or prophylaxis of disorders which are responsive to modulation of hVR, preferably hVR1 or hVR3, receptor activity, in a human patient. By the term "modulation" what is meant is that there will be either agonism or antagonism at the receptor site which results from ligand binding of the compound at the receptor. By the term "modulation" what is meant is that there will be either agonism or antagonism at the receptor site which results from ligand binding of the compound at the receptor excluding the compounds capsaicin, resiniferatoxin, piperine, zingerone, polydodial, warburganal, aframodial, cinnamodial, cinnamosmolide, cinnamolide, isovelleral, scalaradial, ancistrodial, β-acaridial, scutigeral, merulidial, anandamide and capsazepine. hVR , preferably hVR1 and hVR3, proteins have been implicated in disorders of the central nervous system (CNS), gastrointestinal (GI) tract, lungs and bladder and therefore modulation of hVR, preferably hVR1 or hVR3, receptor activity in these tissues will result in a positive therapeutic outcome in relation to such disorders. In particular, the compounds which will be identified using the screening techniques according to the invention will have utility for treatment and/or prophylaxis of disorders such as pain, neuropathic pain, inflammatory pain, chronic pain, post-operative pain, rheumatoid arthritic pain, neuropathies, neuralgia, algesia, neurodegeneration, nerve injury, stroke, ischaemia migraine, IBS, respiratory disorders such as asthma and COPD, urological disorders including diabetic neuropathy, incontinence and interstitial cystitis, and inflammatory disorders. It is to be understood however, that the mention of such disorders is by way of example only, and is not intended to be limiting on the scope of the invention.

The compounds which are identified according to the screening methods outlined above may be formulated with standard pharmaceutically acceptable carriers and/or excipients as is routine in the pharmaceutical art, and as fully described in Remmington's Pharmaceutical Sciences, Mack Publishing Company, Eastern Pennsylvania, 17th Ed, 1985, the disclosure of which is included herein in its entirety by way of reference.

The compounds may be administered via enteral or parenteral routes such as via oral, buccal, anal, pulmonary, intravenous, intraarterial, intramuscular, intraperitoneal, topical or other appropriate administration routes.

The present invention will be further explained, by way of examples, in the appended experimental section. Reference examples are provided.

### Experimental details

### Reference Example A: Identification of related human ESTs (Expressed Sequence Tags) (19) to the rat VR1 sequence by in silico analysis

The full-length rat VR1 amino acid sequence (15) was used as a query sequence using the tBlastn (20) alignment program to identify related human genes in the dbEST (21) and Incyte (Incyte Pharmaceuticals, Inc., 3174 Porter Drive, Palo Alto, California 94304, USA) databases. Several human ESTs were identified and those with similarities greater than 50% selected for further analysis. One of these ESTs was T12251 previously shown to have 68% amino-acid identity and 84% similarity over a region of 70 amino acids (15). Full-length cloning and functional characterisation of the gene represented by this cluster has been completed (30). This gene was denoted hVRL-1 and encoded a protein of 764 amino acid protein that was 48 % identical to the rat VR1 protein. All human ESTs from both databases were clustered to identify overlapping identical ESTs belonging to the same transcript. The GCG package (Wisconsin Package Version 9.0, Genetics Computer Group (GCG), Madison, Wisconsin) and a program developed in house termed ESTBlast (22) were used to build up these clusters. In total, forty-three ESTs derived from different tissue sources and both EST databases were clustered into ten groups, one of these clusters represented hVRL-1. The remaining nine clusters have been named hVRa, hVRb, hVRc, hVRd, hVRe, hVRf, hVRg, hVRh and hVRi. For each EST the tissue source was assigned according to the annotations in the dbEST and Incyte databases. Since no obvious starting codon was present and the cluster sequences were shorter than the rat VR1 transcript none of these clusters were likely to represent a full-length vanilloid receptor transcript. Finally hVRg, hVRh and hVRi collapsed into a single contig. Sequence analysis has shown that these cDNAs are likely to be chimeric. The 5' end has weak similarities with the rat VR1 gene but the 3' end is identical to a DNA binding protein. No more work was pursued with that transcript.

### Reference Example B: Isolation of the human orthologue to the rat VR1 gene (reference examples B1-B4):

### Reference Example B1: In silico assembly of human VR1

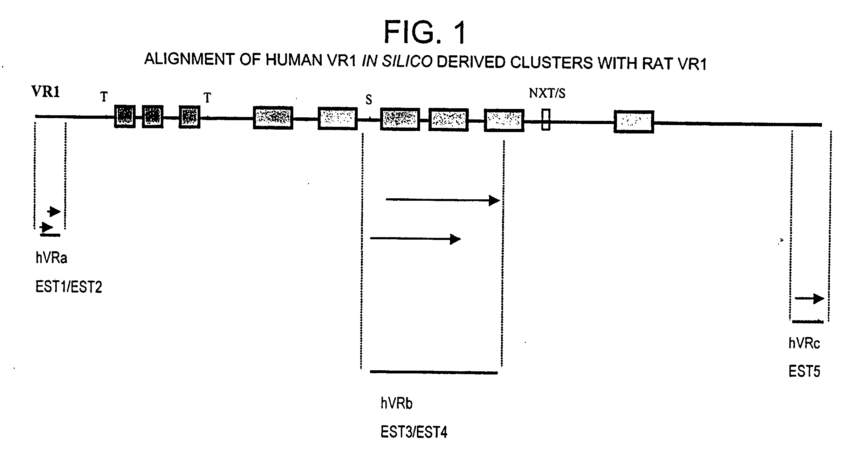

The consensus nucleotide sequences from the ten clusters were searched with the tBlastx program (20) against the rat VR1 sequences to identify the most likely open reading frames. Frame shifts were corrected when the sequence trace files were available. Each cluster was aligned against the rat VR1 amino-acid sequence according to the Blastx results. The Blastx alignment program (20) was used to compare the full-length rat VR1 protein with the amino-acid sequences of the ten clusters. The three clusters with the highest homology, displayed in figure 1, were aligned with the rat VR1 gene.

Cluster hVRa shared a high homology (70% identity and 75% similarity over a stretch of 107 amino acids) with the 5' of the rat VR1 sequence but did not seem to have a potential start codon. It contained two ESTs (EST1 and EST2) derived from the same tissue, bladder, and from the same patient. These two ESTs were selected for further investigation since this cluster was the most 5', had high homology with rat VR1 and the bladder tissue could be contaminated with sensory neurones. Both cDNA clones were ordered but only clone EST1 was received as EST2 failed the recovery procedure.

Cluster hVRb composed of two EST's (EST3 and EST4), with 89% identity and 92% similarity over 90 residues, showed the highest degree of homology to the rodent sequence. The overlap between both sequences was located towards the middle of the gene.

hVRc (EST5) also while having high homology (71 % identity and 75% similarity over 65 residues) with rat VR1 was closely related to the C-terminus of the rat protein sequence.

### Reference Example B2: Sequencing of clones

All DNA sequences were determined by automated DNA sequencing based on the dideoxy chain-termination method using the ABI 373A / 377 sequencers (Applied Biosystems). Sequence-specific primers were used with the 'Big-Dye' Terminator Cycle Sequencing kit (Applied Biosystems). The nucleotide sequence was analysed using programs from the University of Wisconsin Genetics Computer Group package.

More specifically when sequencing an EST clone, the following protocol was adopted. The EST1 clone was grown using standard procedures and DNA was isolated using Qiagen columns. SP6 (5' ATTTAGGTGACACTATAG) and T7 (5' TAATACGACTCACTATAGGG) primers flanking the cloning site were used to sequence both ends. Plasmid DNA (0.6 pmol) was used with 10.0 pmol of each primer for the dye terminator reaction. The SP6 end corresponded to the *in silico* derived EST sequence (identical to EST1). The T7 end did not have homologies with VR1 nor did it possess a long open reading frame or a polyadenylation motif. The size of the insert was determined by enzyme digestion of the DNA with the endonucleases Notl and EcoRI and calculated to be approximately 3kb.

Plasmid DNA (50ng) was used to amplify the insert by Polymerase Chain Reaction (PCR) with T7 and SP6 as primers. The PCR conditions included an initial hot-start at 94°C for 2 minutes, followed by 35 cycles at 94°C for 45 seconds, 50°C for 45 seconds and 72°C for 1 minute and terminated by 5 minutes at 72°C. The resulting PCR amplicon was separated on a 1.2% agarose gel and shown to be of ~3kb in size.

To fully sequence the PCR product the nuclease-Bal-31 technique was used where both strands of duplex DNA are degraded from both ends (23). After ethanol precipitation of the PCR product, the pellet was re-suspended in 30ml of 1X Bal-31 buffer (add buffer composition). A time-course digest with 2 units of Bal-31 enzyme (Roche Molecular Biochemicals) was carried out with 12 time points taken over 90 minutes (30 seconds, 1, 2, 3, 5, 7, 10, 15, 25, 45, 75 and 90 minutes). Three pools were made respectively from digests 1 to 4, 5 to 8 and 9 to 12. Each pool was blunt-ended and sub-cloned into the pCR-Script SK (+) plasmid from Stratagene at the Srfl site. After transformation, 16 colonies from each pool were screened by PCR with the flanking Reverse (5' GGAAACAGCTATGACCATG) and M13-20 (5' GTAAAACGACGGCCAGT) primers. The amplicons of 6 positive colonies per pool were subjected to direct sequencing (24) using the T3 (5' AATTAACCCTCACTAAAGGG) and T7 primers. The DNA sequences obtained were assembled using the GCG package, translated and aligned against the rat VR1 gene using the Blast tools. After analysis, the 3079bp amplicon was shown to have 2 introns of 603bp and 1221bp. The latter intron was located at the 3'end of the PCR product. The coding sequence covered 1255 bp and was separated by the former intron. Therefore the clone EST1 was likely to be a partially spliced and incomplete cDNA.

The clone belonging to cluster 1b (EST3) and derived from a kidney cDNA library was ordered and sequenced using the Bal-31 technique. After assembly of the sequences using the GCG package an identical overlap was identified with the DNA sequence of the cluster hVRc. Moreover a 3'end with a polyadenlyation signal and tail was identified. The complete sequence of the combined hVRb Bal-31 derived sequence and hVRc was 2063 bp (1020 bp of coding and 1043 bp of 3' untranslated sequence).

### Reference Example B3: Amplification of the middle section of HVR1 using the Polymerase Chain Reaction

We formulated the hypothesis that both sequences (hVRa and hVRb/c) were part of a common transcript. If the human and rat VR1 were going to be similar, the 2 contigs should be separated by a gap of approximately 275bp. Primers were designed on both sides of the gap to amplify mRNA from brain tissues in order to clone the gap. A smear was obtained with the sense primer (5' TCTACTTCGGTGAACTGCCC) and antisense (5' ACGGCAGGGAGTCATTCTTC). For specificity 50ng of the PCR product were amplified with the nested sense (5' CTGCAGAACTCCTGGCAGA) and antisense (5' GTCACCACCGCTGTGGAAAA) primers. The 900bp nested amplicon was sequenced and shown to be identical to hVRa at one end and hVRb/c at the other end. The middle part of the PCR product was homologous to the rat VR1 sequence. This region corresponded to 91 amino acids. When the sequences of hVRa, hVRb/hVRc and the internal amplicon are combined the total length of the Open Reading Frame (ORF) is 824 amino acids followed by a 3' untranslated sequence of 1043 bp. The human amino acid sequence is 87% identical to the rat sequence over that part of the coding region. This sequence was termed hVR1 because of its high degree of identity with the rat VR1 sequence.

### Reference Example B4: Isolation of the 5' Terminus of hVR1 by PAC isolation

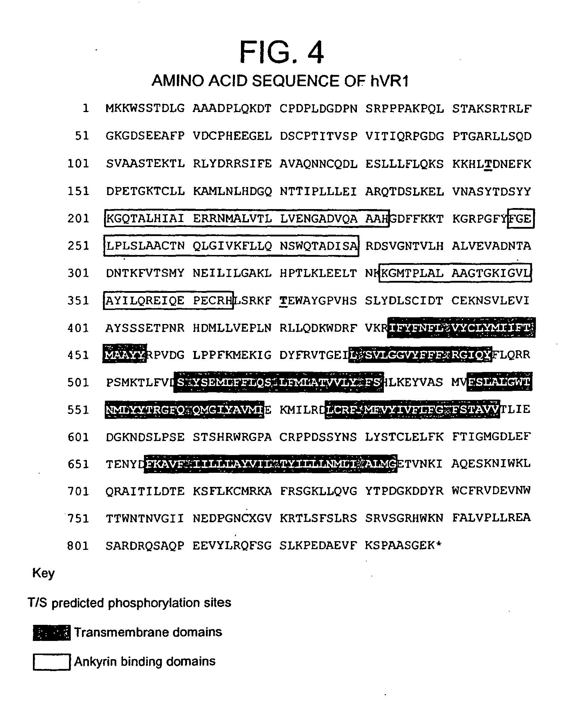

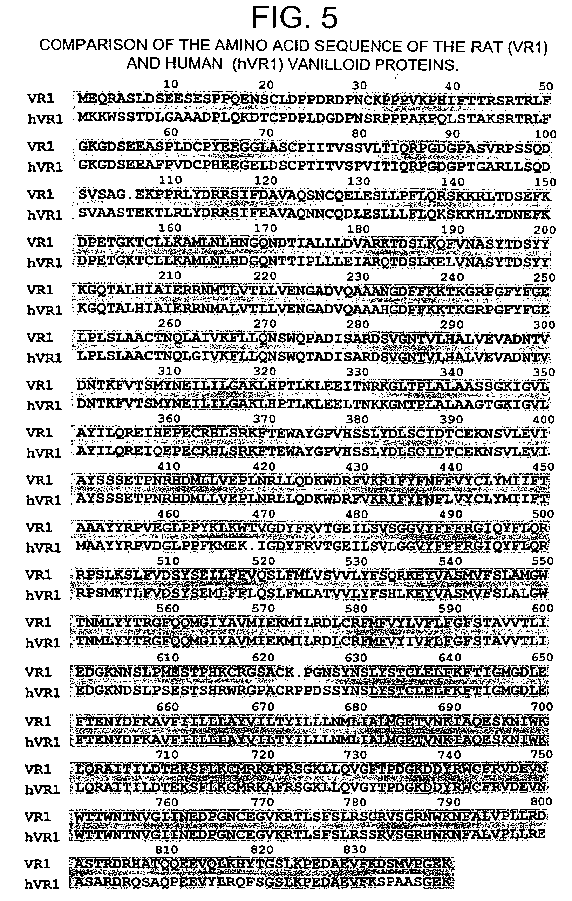

Since no start codon was identified at the 5' end an additional strategy was designed to identify the full-length sequence. Two primers, sense (5' TCCTCTGGCTTCCAACCCGTT) and antisense (5' GAACTGGGCAGAAAGTGCCT) were designed to amplify a 150bp product from the first intron mentioned in reference example B2. A P1 Artificial Chromosome (PAC) genomic clone (25) was isolated by PCR screening of a PAC library (Genome Systems, St Louis, Missouri). PAC DNA was recovered by using standard plasmid isolation protocol (26). An anti-sense primer was designed (5' CTGGAGTTAGGGTCTCCATCC) to sequence the genomic clone towards the potential 5' end of the gene. An open reading frame with a starting codon was identified. The gene structure was confirmed by using the GenScan software (27). The complete gene has a nucleotide sequence of 2517bp (figure 2) and encoded a 839 amino acid protein (Figures 3 and 4). The gene was named hVR1. Multiple alignment of the amino acid sequence of hVR1 and rat VR1 shows a remarkable degree of identity and similarities between both sequences (figure 5). The rVR1 and hVR1 amino acid sequences are 86% identical. Moreover after protein analysis 6 trans-membrane domains and 3 ankyrin binding domains were identified in hVR1 as in the rat VR1 gene.

### Example 1: Full-length Amplification of HVR1 from human DRG and assembly into cloning vectors

HVR1 was PCR amplified in three sections from human DRG template. The 5' fragment was amplified using a sense primer encoding a Notl site and a strong Kozak motif followed by gene specific sequence (5' GTCATAGCGGCCGCGCCGCCACCATGAAGAAATGGAGCAGCAC) and an antisense primer (5' AGGCCCACTCGGTGAACTTC). The thermo-cycling conditions used for this amplification included a hot start at 94°C for 4 mins, followed by 35 cycles of 94°C for 1 min, 54°C for 1 min and 72°C for 1 min. A final extension step of 72°C for 5 min completed the reaction. The resulting PCR products were separated on a 2% agarose gel and cloned into pCR® II-TOPO according to the manufacturers instructions supplied with the TOPO^{TM} TA Cloning® kit (Invitrogen). The middle section of hVR1 was PCR amplified using the sense primer: 5' GACGAGCATGTACAATGAGA and antisense primer: 5' GTCACCACCGCTGTGGAAAA. The cycling conditions included a hot start at 94°C for 4 mins, followed by 35 cycles of 1 min at 94°C, 56°C and 72°C. A final extension step of 72°C for 5 min completed the reaction. A band of approximately 870 bp was excised from a 2 % agarose gel and cloned as detailed by the TOPO^{TM} TA Cloning® kit into the vector pCR2.1® -TOPO. Finally the 3' end was PCR amplified with the sense primer: 5' TGTGGACAGCTACAGTGAGA and the antisense primer: 5'TGCACTGAATTCGAGCACTGGTGTTCCCTCAG which encoded an EcoRI site for cloning. The PCR conditions included a 90 sec hot start at 94°C followed by 35 cycles of 94°C for 50 sec, 50°C for 50 sec and 72°C for 50 sec. The cycling was completed with a 72°C step for 5 min. PCR products were separated on a 2% agarose gel and cloned into the vector pCR2.1® -TOPO.

Resulting clones for each of the three hVR1-fragments were taken for sequence analysis and separate clones coding a consensus sequence were used in the full length assembly of the gene. The Notl/DraIII (New England Biolabs) digested 5' end fragment ligated together with the middle DraIII/EcoRI fragment into a Notl/EcoRI restricted pBluescript SK (+) vector (Stratagene). Finally, the remaining 3' fragment was introduced into the resulting construct via Mscl and EcoRI restriction sites, a map of the resulting construct is displayed in figure 6A.

Several clones were selected for sequence analysis to confirm that constructs still encoded the hVR1 consensus sequence. These were then digested with Notl/EcoRI and ligated into the mammalian expression vector pCIN5-new (a modified version of pCIN1 (32) having an IVS deletion as well as a 36 bp deletion repositioning the start codon of neomycin phosphotransferase immediately after the upstream EMVC IRES) as illustrated in figure 6B.

### Example 2: Chromosomal Localisation

The primers used to isolate the PAC clone (reference example B4) were selected for PCR on the G3 radiation hybrid panel from Stanford commercially available from Research Genetics (Huntsville, Alabama). The positive lanes and negative patterns were analysed using the public web server at Stanford University (http://www-sghc.stanford.edu). After analysis the hVR1 gene appears to be located on human chromosome 17 around marker SHGC-36073 (lod score=9.55).

### Example 3: mRNA Distribution

The tissue distribution of hVR1 was established by slot-blot hybridisation. RNA was transferred onto a sheet of GeneScreen hybridisation transfer membrane (DUPONT) sandwiched in a slot blotter by suction via a vacuum pump. Once the membrane was rinsed in 2x SSC (3M sodium chloride and 0.3M sodium citrate pH7) for 2 min it was exposed to UV using an Ultraviolet crosslinker (Amersham Life Science) for 1 min at 15000uW/cm² thus enabling cross-linkage of the RNA onto the membrane. The amounts of RNA on the blot are unknown. The probe was obtained by PCR amplification of a 260 bp product of the coding region of hVR1 with the following two primers: 5' TGTGGACAGCTACAGTGAGA and 5' GTGGAAAACCCGAACAAGA. Membranes were hybridised for 4 hr shaking at 60°C in a 10% dextran sulphate, 1% SDS (sodium dodecyl sulphate) and 1 M NaCl solution. The probe was labelled with [α32P]dCTP (Amersham) using the Rediprime^{TM}DNA labelling system (Amersham), so as to obtain approximately 500,000cpm of the labelled probe per ml of prehybridisation solution. Briefly 100ng of probe was boiled for 3 minutes (denaturization) and then cooled on ice for 2 minutes in a total volume of 45µl. This was added to the labelling tube from the kit together with 3µl of 32P dCTP followed by an incubation at 37°C for 30 minutes. 400µl of Herring Sperm DNA (Sigma) at a concentration of 8µg/ml was added to the labelled probe and heated at 99°C for 3 minutes followed by rapid cooling on ice. The labelled probe was added and mixed well in pre-hybridisation solution. The membranes were hybridised overnight at 55°C.

The membranes were then washed, first at room temperature in 2xSSC and 1% SDS for 5 minutes, followed by 2x SSC and 1% SDS for 30 min at 50°C. If necessary further washes with 1 x SSC and 0.5% SDS or 0.1 xSSC and 0.1 % for 30 mins at the same temperature were carried out. The membranes were then exposed to Scientific Imaging Film AR (Kodak) using intensifying screens at -70°C overnight and the film developed.

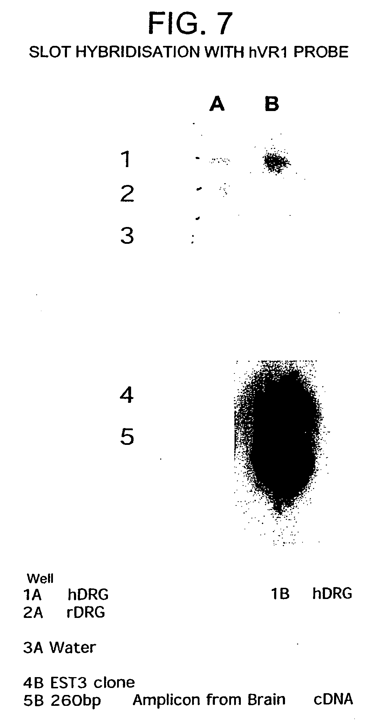

The results are shown on figure 7. Strong signals were observed with the positive controls (slots 4B and 5B). Signals are detected on the human DRG slots (1A and 1B). No signals were detected with the water control (slot 3B). Three multi-tissue northern blots (Clontech) with a wide range of tissues have also been hybridised with the same probe, however no signals were detected. RT-PCR was performed on various tissues with the primer combination used to amplify the probe. A strong band was detected in DRG RNA. Taken together these hybridisations suggest that hVR1 is specifically expressed in neuronal tissue and DRG in particular.

### Example 4: Design and production of Anti-hVR1 Antibody

The peptides CHIFTTRSRTRLFGKGDSEEASC (peptide68) and CGSLKPEDAEVFKDSMVPGEK (peptide69) were synthesised by standard solid phase techniques and purified by gel filtration chromatography. These peptides were conjugated via their Cys residues to the carrier protein, Tuberculin PPD (purified protein derivative) using sulpho-SMCC (sulfosuccinimidyl 4-[N-maleimidomethyl]-cyclohexan-1-carboxylate). Rabbits, previously sensitised to Bacillus Calmette Guerin (BCG), were inoculated with the resulting conjugates emulsified in incomplete Freund's adjuvant at approx monthly intervals. Serum was prepared from blood samples taken 7 days after each immunisation. The specific antibody response was followed by indirect enzyme-linked immunosorbent assay (ELISA) using free peptide as antigen. Immunoglobulins were purified from high titre sera using immobilsed peptide affinity columns (sulpholink Pierce). Rabbits designated M143, 144 and 145 received peptide68 conjugate, rabbits M146, 147 and 148, peptide69 conjugate.

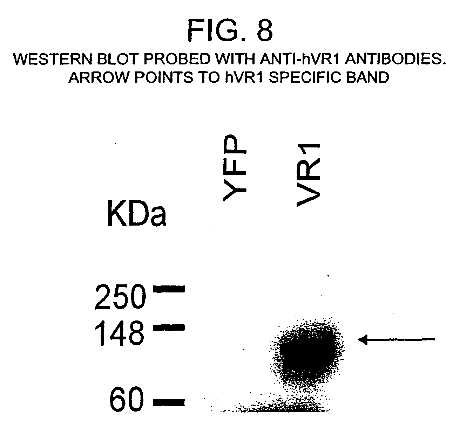

The antibodies have been validated by specific staining of the recombinant protein expressed in HEK293 cells. Whole cell lysates were prepared in Sample Buffer (4 ml dH₂O, 1 ml 0.5 M Tris-HCl, pH 6.8, 0.8 ml glycerol, 1.6 ml 10 % w/v SDS, 0.4 ml 2-β mercaptoethanol and 0.2 ml of 0.05 % w/v bromophenol blue) and proteins separated by SDS-PAGE and transferred to a nitrocellulose filter by electroblotting. Following incubation with the antisera, bound immunoglobulins were revealed using HRP-conjugated secondary antibodies and enhanced chemiluminescence (ECL) detection. The antisera showed specific binding to a protein(s) of the appropriate molecular weight(s) in extracts of VR1 transfected cells, but not in control extracts, this is illustrated in figure 8.

### Example 5: Insitu localisation of hVR1 using specific antibody

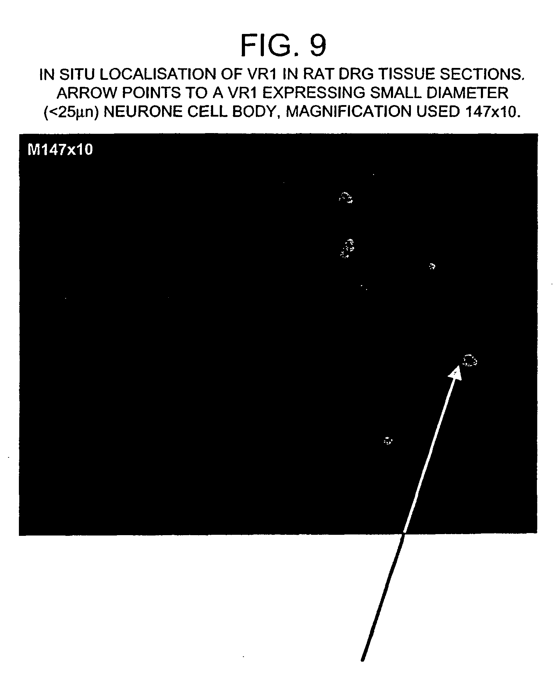

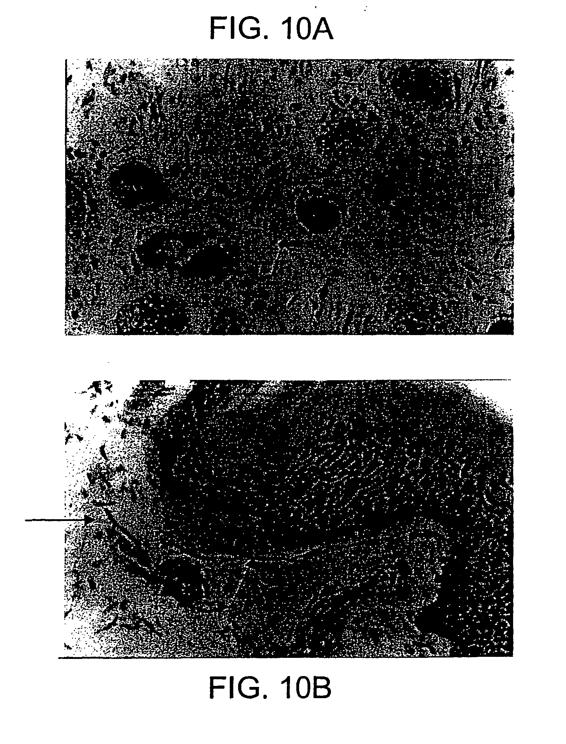

The purified immunoglobulins have been used for immunohistochemical staining of rat DRG tissue sections. Fixed cryosections of DRG were incubated with antibodies for 48h at 4°C at concentrations between 0.1 to 0.5µg/ml. Following a washing step, bound antibodies were detected by indirect immunofluorescence. The antibodies recognised exclusively small diameter cell bodies of the peripheral sensory neurones as displayed in figure 9. This observation has been extended to human DRG tissues for the anti-peptide68 peptide antibodies demonstrating cross-reactivity with the human sequence as expected. Figure 10A demonstrates labelling of DRG cell bodies with an arrow that points to small diameter neuronal cell body) and in figure 10B the arrow points to labelled neurones innervating human skin.

### Example 6: Mammalian Cell Expression (examples 6a-6b)

### Example 6a: Transient expression of hVR1 in mammalian cells

HEK293 cells were plated onto a 6 well plate, containing poly-I-lysine coated coverslips, at 5 x 10⁴ cells per well. Next day, fresh media was added to the cells (50% confluent). CalPhos Mammalian Transfection Protocol (Clontech, K2051-1) was used for DNA transfection. For each well of cells, solution A was made up containing 8ug hVR1pCIN5, 2µg pEYFP-N1 reporter DNA, 12.4 µl calcium solution and water to 100µl. Solution B (hepes buffered saline) was slowly vortexed while solution A was added dropwise. The mixture was incubated at room temperature for 20 minutes, and then added to cells. The plate was slowly rocked to distribute the solution. The cells were incubated at 37°C for 5 hours, and then washed with phosphate buffered saline. Fresh culture medium was added and the plate was incubated 24-48 hours for functional analysis.

### Example 6b: Stable expression of hVR1 in mammalian cells

HEK293 cells were plated onto a 6 well plate at 1 x 10⁵ cells per well. Next day, fresh media was added to the cells (50% confluent). CalPhos Mammalian Transfection Protocol (Clontech, K2051-1) was used for DNA transfection. For each well of cells, solution A was made up containing 2µg hVR1pClN5, 12.4µl 2M calcium solution and water to 100µl. Solution B (hepes buffered saline) was slowly vortexed while solution A was added dropwise. The mixture was incubated at room temperature for 20 minutes, and then added to cells. The plate was slowly rocked to distribute the solution. The cells were incubated at 37°C for 5 hours, and then washed with phosphate buffered saline. Fresh culture medium was added and the plate was incubated 48 hours at 37°C, 5% CO₂. Cells were harvested into 100mm dishes in selection medium containing 800µg/ml geneticin. Cells were then incubated and fed at 4 day intervals. In total around 10 days selection is required for each single cell to multiply into a visible clone. Well-separated clones were each picked (with a gilson tip) into separate wells of a 96 well plate, containing maintenance medium (400µg/ml geneticin). Cells were expanded into flasks for freezing stocks and functional analysis. Stable cells may be plated at 1 x 10⁵ cells onto poly-l-lysine coated coverslips in 6 well plate, for calcium imaging next day.

### Example 7: Functional Analysis of hVR1 (examples 7a-7c):

### Example 7a: Electrophysiology using patch clamp methods

The activation of human VR-1 channels transiently expressed in HEK293T cells by capsaicin was investigated. Cells grown on poly-L-lysine-coated glass coverslips were placed in a recording chamber (0.5ml) and superfused with extracellular solution (2ml min⁻¹). The extracellular solution contained: NaCl (140mM), KCI (5mM), MgCl2 (2mM), CaCl2 (2mM), 4-(2-hydroxethyl)-1-piperazineethanesulphonic acid (HEPES, 10mM) and glucose (10mM). The pH was adjusted to 7.4 with NaOH and osmolarity ranged from 310-320mOsm I⁻¹. Patch pipettes (borosilicate glass) were pulled using a Suffer P-97 electrode puller. The pipettes were filled with an internal solution consisting of: CsCl (140mM), ethylene glycol-bis(β-aminoethyl ether) *N,N,N;N'*-tetra acetic acid Cs salt (Cs-EGTA, 5mM) and HEPES (10mM). The pH was adjusted to 7.25 using CsOH and the osmolarity ranged from 275-290 mOsm. When filled with this internal solution, patch electrodes had resistances of 2-5 MΩ. Currents were recorded using standard whole-cell voltage clamp recording techniques (31) at room temperature (21-23°C) using an Axopatch 200A amplifier and signals were sampled at 2 or 0.1 kHz. The majority of series resistance errors (80-85%) were minimized with compensation circuitry. Membrane potentials were not corrected for junction potentials (<4 mV). Voltage pulses and data collection were performed on-line using pClamp8 software (Axon Instruments) interfaced with amplifiers. Membrane potentials were maintained at -60mV between protocols.

Capsaicin or capsazepine (CPZ) were applied, using a 'fast-flow sytem', directly onto the recording cell (<1s to equilibrate). The effects of capsaicin were measured either by application during constant recording while holding the membrane potential at -60mV to elicit an inward current, or applying voltage ramps (-100 to +60mV) in the absence and presence of capsaicin. Similarly both these methods of recording currents evoked by the application of capsaicin were used to demonstrate the blockade by the antagonist (CPZ).

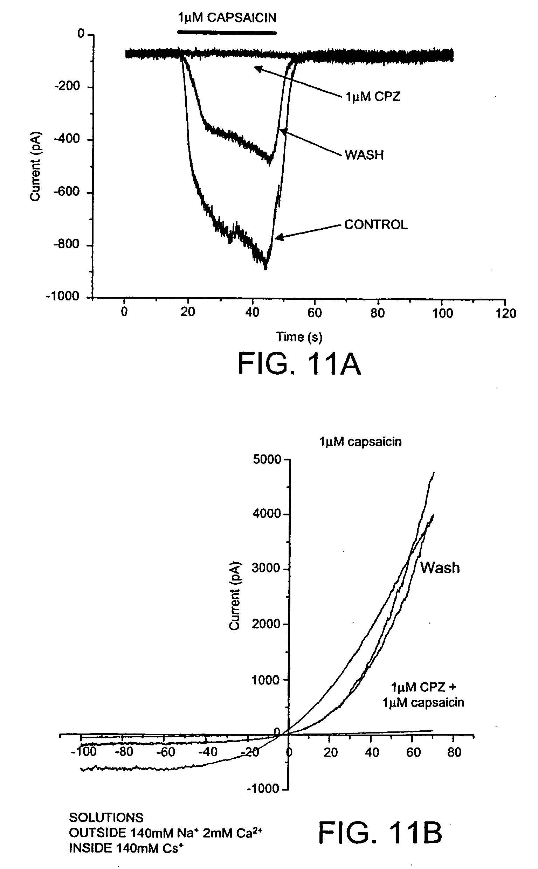

Figure 11A reveals that application of capsaicin (1 µM), on human VR1 channels transiently expressed in HEK293T cells, produces an inward current when the membrane was held at a potential of -60mV. This response was abolished by 1µM CPZ and the blockade was partially reversible.

In the presence of 1 µM capsaicin, voltage ramps (-100 to +70mV) produced a current-voltage relationship demonstrating a substantial outward rectification. Addition of 1µM CPZ completely blocked the current (figure 11 B). Again, only partial recovery was observed, especially for the inward currents evoked by negative potentials.

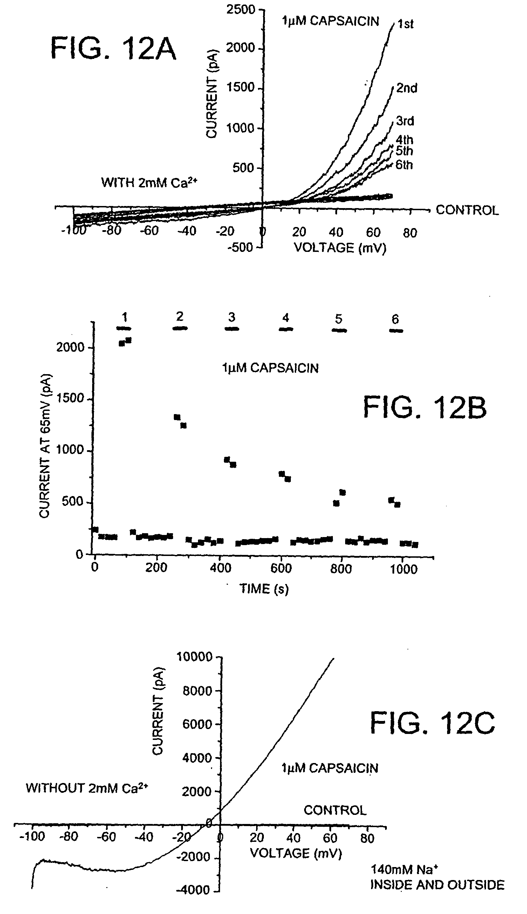

Capsaicin-induced desensitisation of human VR-1 channels in the presence of 2mM external calcium is illustrated in figure 12. Voltage ramps (-100 to +70) were applied and the addition of capsaicin (1µM) evoked an outwardly rectifying current. Repeated additions of capsaicin resulted in a progressive 'rundown' in the size of the response (figure 12A). Figure 12B shows a plot of the current elicited at a potential of +65mV against time illustrating the 'rundown' in current amplitude. Voltage ramps were applied every 20s and capsaicin added at 2min intervals for approximately 40s. By the 6th addition the current had reduced about 4-fold.

When the external calcium was replaced with 5mM EGTA the size of the current increased dramatically (figure 12C). However, when calcium was re-applied to the external solution, the current evoked by capsaicin (1µM) was approximately equivalent to that of the 6th addition shown in (figure 12A).

### Example 7b: Calcium Imaging with HEK293 expressing HVR1

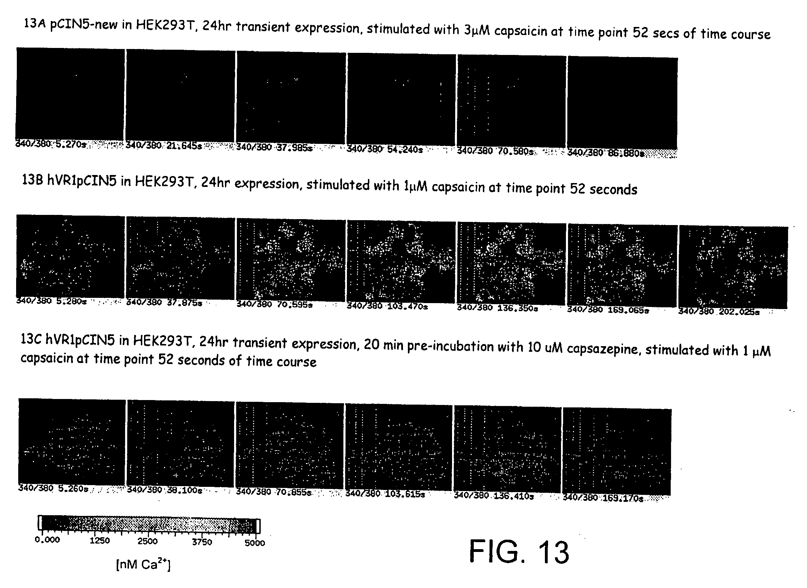

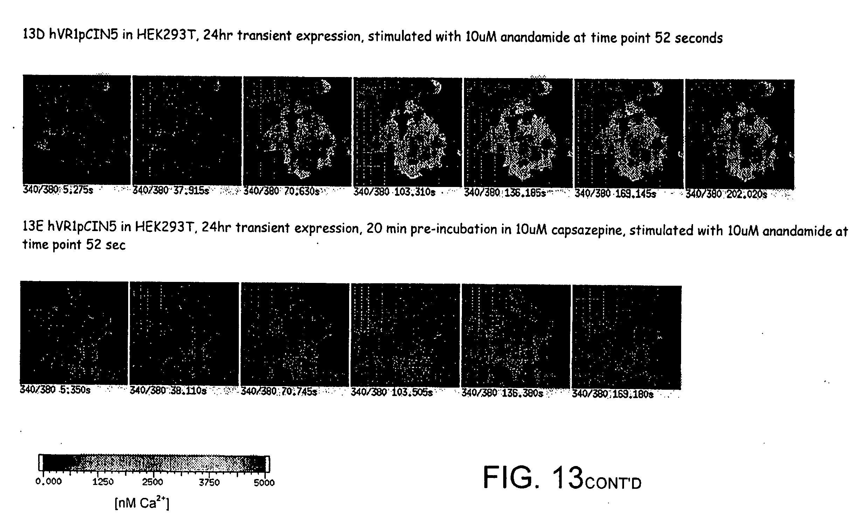

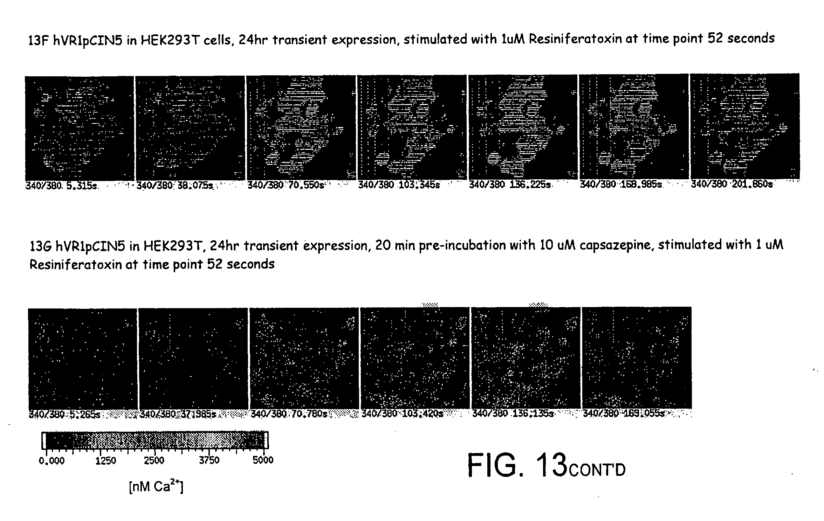

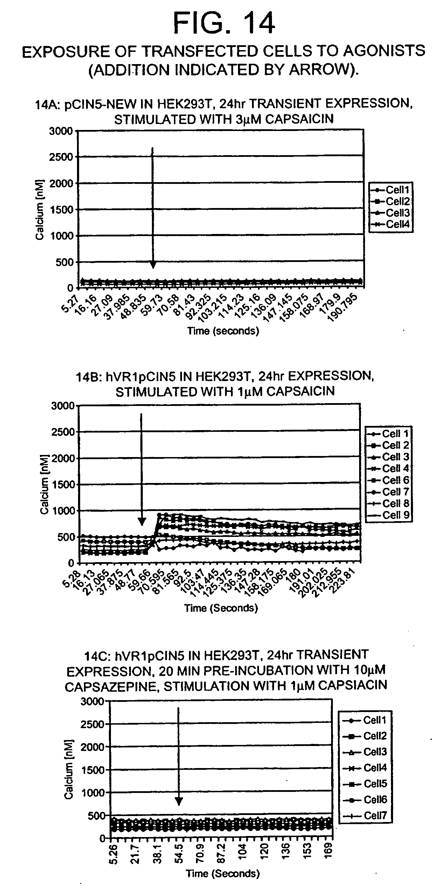

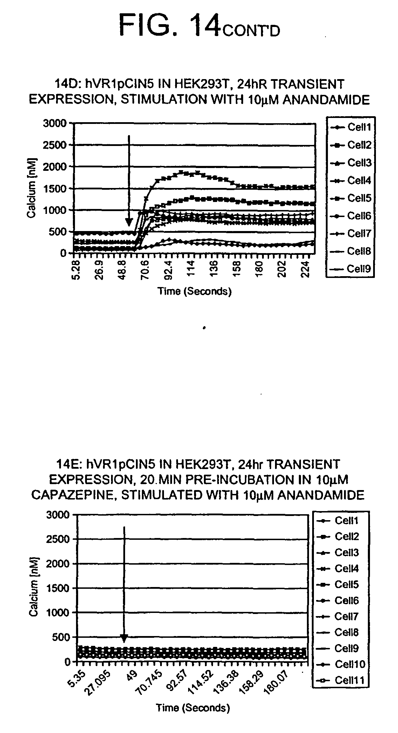

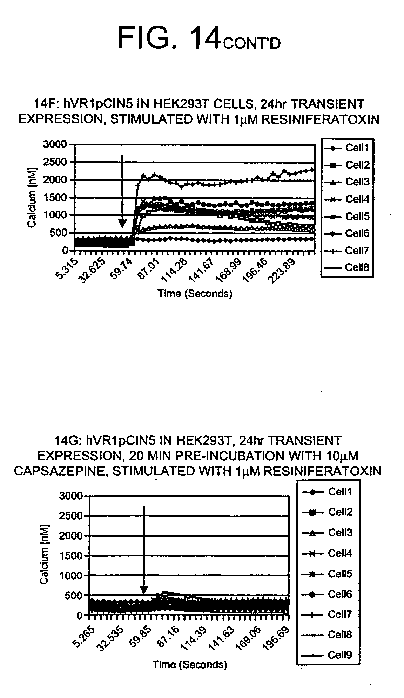

HEK293 cells expressing hVR1 transiently or stably, were plated onto poly-I-lysine coated cover slips at 1 x 10⁵ cells per well. They were analysed on the following day by calcium imaging (QuantiCell 700, Applied Imaging). On the day of experiment, WASH buffer was prepared by adding CaCl₂ to extracellular medium (ECM) to a final concentration of 2mM, (ECM contains 125mM NaCl, 5mM KCl, 2mM MgCl₂, 0.5mM NaH₂PO₄, 5mM NaHCO₃, 10mM Hepes, 10mM glucose, 0.1% BSA, pH7.4). The calcium sensitive dye solution was prepared by adding 50µl 5% pluronic F-127 in DMSO (Molecular Probes) to a vial of fura2-AM (Molecular Probes). After mixing, 20µl of the fura2-AM solution was added to 10ml WASH. 1.5 ml was then added to cells, which were then incubated at 37°C for 30 minutes. The plate was washed three times with WASH. 1ml WASH was added and stored in dark. Agonists and antagonists were prepared in WASH at 5x their required assay concentrations. The reagents and assay temperature was kept at 37°C. For the transiently transfected cells, the YFP reporter DNA fluorescence (490nm excitation) was used to identify the transfected cells. Cells were initially imaged in 400µl WASH (or 300µl WASH plus 100µl antagonist e.g. capsazepine). After approximately 1 min, 100µl agonist (e.g. capsaicin, anadamide or resiniferatoxin) at 5 x the desired concentration was added to give final 1 x concentration. A sequence of images (340/380nm excitation) were taken to monitor calcium influx response in cells before (30-60 secs), and after the addition of agonist (2-5 mins). Figure 13 displays time courses taken for each of the tests set up to look at the affect of the different agonists mentioned above in the presence or absence of the rat VR1 antagonist, capsazepine. The Imager also plots graphs of respective calcium concentration (nM) versus time (seconds) as shown in figure 14. After the addition of agonist (e.g. capsaicin, indicated by the vertical arrow on graph), the cells expressing hVR1 are stimulated to influx calcium. This is shown by the appearance of peak on the trace. The peak height correlates with hVR1 expression level. Varying levels of expression is some times seen depending on which cells are selected for the graph. Similar experiments may be accomplished to examine the response of protons and heat.

### Example 7c: Use of a FLIPR assay with VR1

FLIPR (Fluorometric Imaging Plate Reader) is a high throughput fluorescence-based drug discovery tool for functional cell analysis. Intracellular calcium is monitored with the calcium sensitive dye, fluo3-AM. HEK293 cells stably expressing rat VR1 were plated into a 96 well, poly-I-lysine treated FLIPR plate at 3 x 10⁴ cells per well. On the following day, the plate was processed for FLIPR. FBP buffer was prepared (15µM Probenecid (calcium ATPase pump blocker) in 1x FLIPR buffer (145mM NaCl, 5mM KCI, 1mM MgCl2, 2mM CaCl₂, 10mM glucose, 20mM Hepes). FBP buffer pH was then adjusted to 7.4 with NaOH. 400µl DMSO was added to a vial of fluo3-AM (Cambridge Bioscience, F-1241). The fluo3-AM solution was incubated at 37°C for 10 min and vortexed. LOAD was prepared by adding 20µl of fluo3-AM solution and 20µl 20% pleuronic F-127 in DMSO (Cambridge Bioscience, P-3000) into 10 ml FBP. The 96 well plate containing cells was flicked off to remove cell medium. 100µl LOAD was added per well. Cells were then incubated at 37°C for 60 minutes. Capsaicin (a rVR1 agonist) and capsazepine (CPZ, a rVR1 antagonist) were prepared at 10x the desired final assay concentrations in FBP. The plate was flicked to remove LOAD from cells, and 180µl FBP was added per well. The FLIPR machine added 20µl capsaicin per well to give a final 1 x concentration. Cells were monitored for 70 seconds after agonist addition. The FLIPR traces (fluorescence change (counts) versus time (seconds)) were produced for each well. Peaks indicate capsaicin-gated calcium influx, by cells expressing rVR1. The peak height correlates with the rVR1 expression level. To measure antagonism of the VR1 response 20µl 10x antagonist CPZ was added into wells to give a final 1x concentration. The plate was incubated for 15 minutes at room temperature prior reading in the FLIPR. The FLIPR traces recorded for each well show that the peak heights are reduced in cells pre-incubated in CPZ. The same FLIPR assay may be used to monitor the response of human VR1 on exposure to agonists and antagonists.

### Example 8: Example of a screen using human VR1.

FLIPR assay technology may be utilised to screen for hVR1 modulators according to the procedure described in figure 15. Human VR1 may be gated with protons, capsaicin or heat.

### Reference Example C: Identification and partial characterisation of additional human vanilloid receptors (referenence examples C1-C3):

### Reference Example C1: Identification and characterisation of a novel vanilloid-like receptor, hVR3

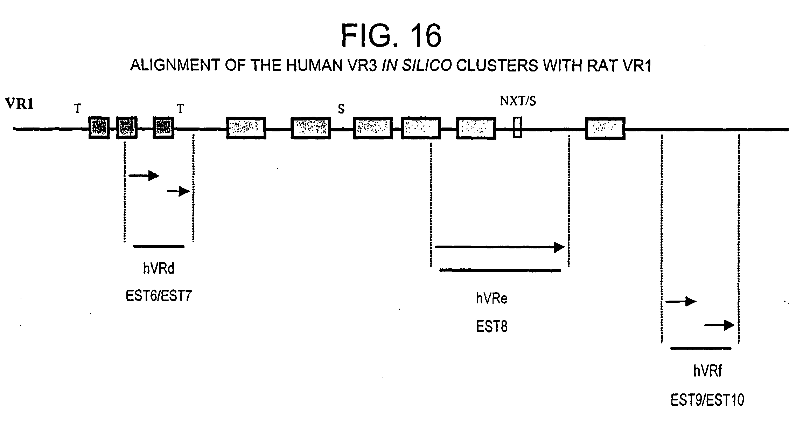

ESTs belonging to the remaining clusters were characterised by *in silico* cloning (reference example A). The following clones were used during this process: -EST6/EST7 (hVRd), -EST8. (hVRe), - EST9/EST10. (hVRf). These EST clusters have been aligned with rat VR1 in figure 16, note that this diagram is not to scale.

### Reference Example C2: Sequencing of clones

Further sequencing, as detailed in reference example B2, and *in silico* cloning, enabled clusters hVRd, hVRe and hVRf to collapse forming a single contig of 583 amino acids. This sequence was named hVR3 and has 49 % identity with the rat VR1 sequence. It was unlikely that this single contig was a full-length vanilloid receptor transcript as no obvious starting codon was present and it was shorter than the rat VR1 transcript.

### Reference Example C3: Identification of the 5' terminus of hVR3

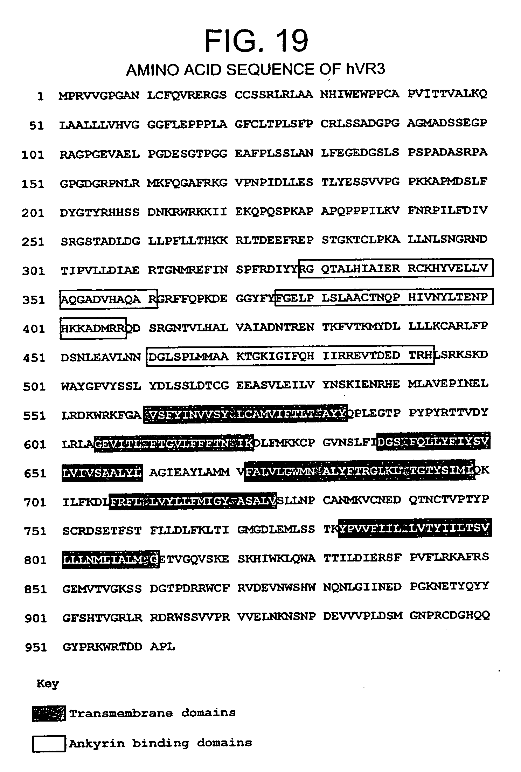

Two primers (sense primer 5' ATGGCCACCAGCAGGGTTAC and antisense primer 5' TCTGCCAGGTTCCAGCTG) designed to PCR amplify an amplicon stretching the 3' end of hVR3 and its 3'utr were used to isolate a genomic PAC clone (Genome Systems. St Louis, Missouri). The hVR3 specific PAC clone was then used as template to generate a library. This was achieved by sonicating 6µg of Qiagen purified PAC construct, size selecting fragmented DNA of 500-2000bp. These resulting fragments were then blunt ended and cloned into the vector pCR® -Blunt as detailed in the manufacturers protocol supplied with the Zero Blunt^{TM} PCR cloning kit (Invitrogen). Clones were then sequenced (reference example B2) to identify the complete 5' end of the hVR3 transcript. The full-length nucleotide sequence of the hVR3 gene is displayed in figure 17. Figure 18 illustrates both nucleotide and encoded amino acid sequence of the human VR1 and figure 19 depicts the amino acid sequence of the hVR3 gene with shaded regions denoting predicted trans-membrane regions (boxed) and the ankyrin binding domains (unboxed).

### Example 9: Full-length Amplification of hVR3 from human kidney template

Human kidney was used as a source of template for the PCR amplification of hVR3. Primers used for amplification were designed to isolate the gene in three fragments. Primers designed to isolate the 5' end included a sense primer encoding a Notl site and a strong Kozak motif followed by gene specific sequence (5' GTCATAGCGGCCGCGCGCCACCATGCCCAGGGTAGTTGGAC and antisense primer (5' CACCTCTTGTTGTCACTGGA). The PCR conditions used were a hot start at 94°C for 4 mins, followed by 35 cycles of 94°C for 1 min, 56°C for 1 min and 72°C for 1 min and finally one cycle at 72°C for 5 min. The resulting PCR products were separated on a 2% agarose gel and cloned into pCR® II-TOPO according to the manufacturers instructions supplied with the TOPO^{TM} TA Cloning® kit (Invitrogen). The middle fragment was PCR generated using sense and antisense primers 5' CAAATCTGCGCATGAAGTTCCAG and 5' GCCACGAGAAGTTCCACGTAGTG respectively in the presence of 5% DMSO. PCR thermo-cycling required 35 cycles of 1 min at 94°C, 58°C and 72°C for successful amplification of the fragment which was then excised from a 2% agarose gel for cloning into the pCRII® -TOPO vector. Finally the 3' fragment was amplified with a sense primer 5' GCTGCTCCCATTCTTGCTGA and an antisense primer 5' TGCACTCTCGAGAAATGAGTGGGCAGAGAAGC encoding a Xhol restriction site. This fragment was successfully amplified using a hot start at 94°C for 4 min followed by 35 cycles of 94°C for 50 sec, 48°C for 50 sec and 72°C for 2 min. The cycling was completed with a 72°C step for 5 min. The amplified fragment was excised from a 2% agarose gel and clone into the pCRII® -TOPO vector.

Resulting clones for each of the three PCR generated hVR3-fragments were taken for sequence analysis and separate clones coding a consensus sequence were used in the full-length assembly of the gene. The Dralll restriction site of the pBluescript SK (+) vector (Stratagene) was firstly abolished by digestion with Dralll followed by a blunt ending step using T₄ DNA polymerase (New England Biolabs). This modified vector was then restricted to enable the ligation of both a Notl/Ncol 5' fragment and Ncol/ EcoRI middle fragment. Finally, the remaining 3' fragment was introduced into the resulting construct via Dralll and Xhol sites (figure 20A).

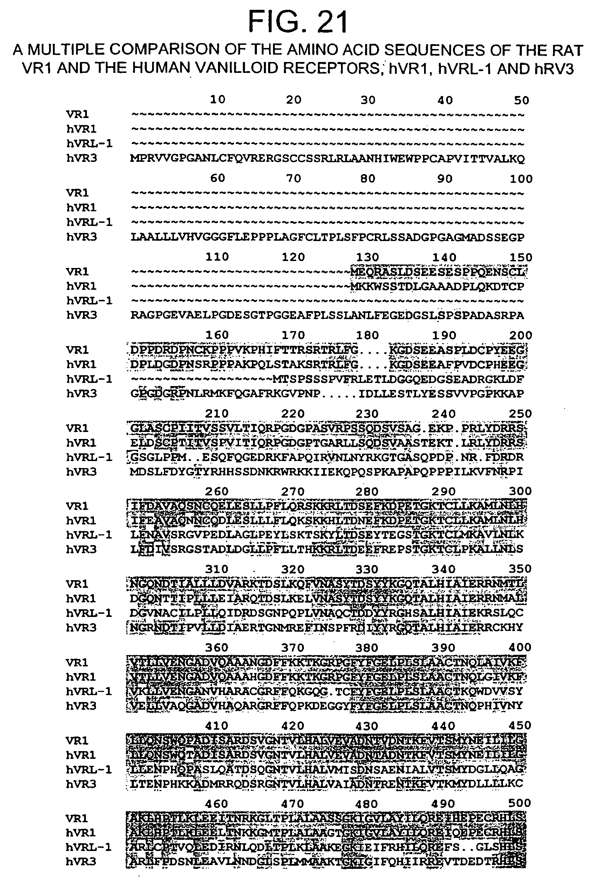

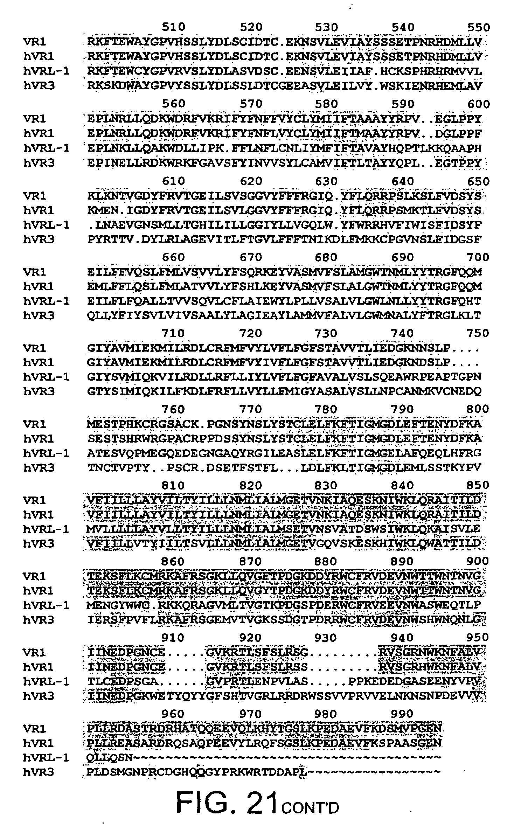

Several clones were selected for sequence analysis to confirm that the constructs still encoded the hVR3 consensus sequence. These were then digested with Notl/Xhol and ligated into the mammalian expression vector pCDNA3.1 (+) (Invitrogen) as seen in figure 20B. The resulting hVR3 consensus sequence is shown in the multiple alignment along with the full-length sequence of hVR1 and the published hVRL-1 in figure 21.

### Example 10: Chromosomal localisation

The 3' terminus, including the 3' UTR sequence of hVR3 was used to design two primers to amplify a product of 360 bp: sense primer 5' ATGGCCACCAGCAGGGTTAC and antisense primer 5' TCTGCCAGGTTCCAGCTG. The G3 radiation hybrid panel from Stanford University (Research Genetics, Huntsville, Alabama) was screened by PCR. The positive and negative lanes were analysed using the public web server at Stanford University (http://www-sghc.stanford.edu). After analysis the hVR3 gene appears to be located on human chromosome 12 around markers D12S177E (lod score=15) and D12S1893 (lod score=14).

### Example 11: mRNA distribution

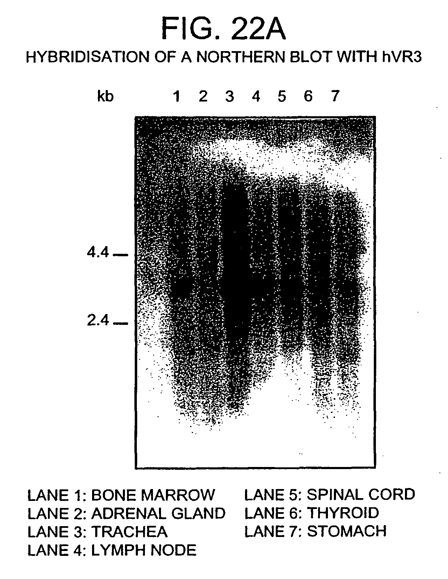

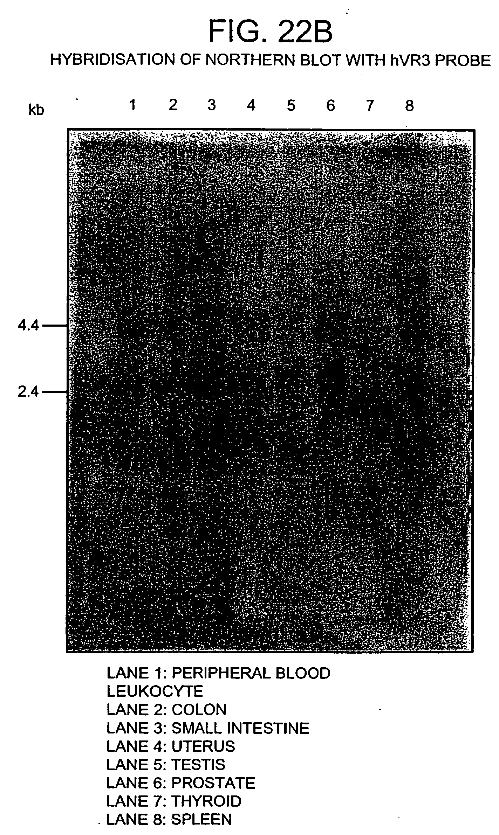

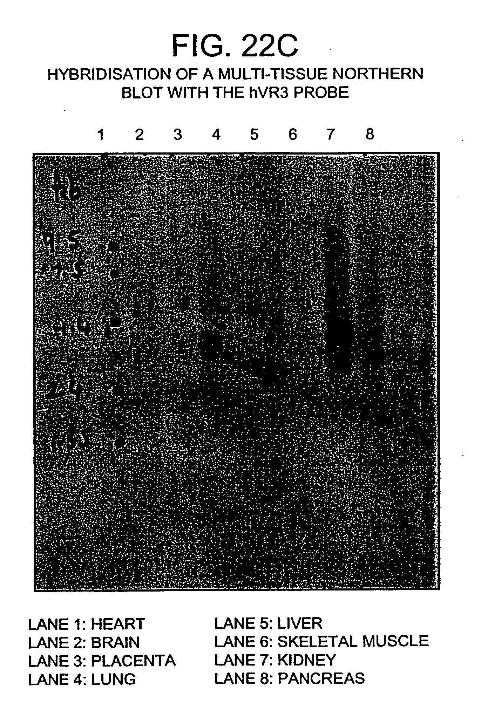

The following primers (5' ACAAGAAGGCGGACATGCGG and 5' ATCTCGTGGCGGTTCTCAAT) were used to obtain a PCR product from the coding region of hVR3. This amplicon was used as a probe on multi-tissue northern blots, the protocol of which is detailed in example 3, to determine the tissue distribution of the gene (figures 22A, 22B and 22C). A transcript of approximately 3.8 kb was detected in the following tissues (the intensities of the signals are indicated in brackets): trachea (very strong), kidney (strong), pancreas (strong), prostate (strong), placenta (strong), bone marrow (weak), adrenal gland (weak), lymph node (weak), spinal cord (weak), thyroid (weak), stomach (weak), lung (weak) and liver (weak).

Since these commercial blots (Clontech, Palo Alto, California, USA) should have the same amount of RNA it is interesting to note the very strong signal in the trachea lane (figure 22A). This could indicate the potential of hVR3 as a target for respiratory pathologies. It was shown by RT-PCR with the primer combination used to produce the probe that the gene is not expressed in DRG.

### Example 12: Riboprobe generation for the in situ localisation of hVR3

The same probe, which was specific to hVR3 in Northern blot analysis (example 11), was used to generate a riboprobe. This hVR3 specific probe was cloned into the T7 and SP6 encoding pCRII® -TOPO vector (Invitrogen). This construct was then used in the *in vitro* transcription of DIG labelled RNA strands from the vectors promoters as described in the manufacturers instructions as detailed in the DIG RNA labelling kit (Roche Molecular Biochemicals). This riboprobe may be used to identify the cellular localisation of hVR3 present in tissues such as trachea, lung, pancreas, prostate, placenta and kidney.

### Example 13: Mammalian Cell Expression of hVR3

Expression of hVR3 may be accomplished by transfecting a mammalian cell line such as: HEK283T, HEK293, CHO, COS, HeLa and BHK. A detailed method for both transient and stable transfection is detailed in example 6.

### Example 14: Functional Analysis of hVR3

The functional analysis of hVR3 may be studied using the electrophysiology, calcium imaging and FLIPR methods as detailed in examples 7a to 7c.

### Example 15: Example of a drug screen using human VR3.

A stable cell line expressing hVR3 may be used in a drug screen such as a selectivity screen using test compounds that have been identified to have an agonistic or antagonistic action on hVR1. FLIPR assay technology may be utilised to screen for hVR3 modulators as proposed in figure 15.

### References

1. Szallasi, A. and Blumberg, P.M (1993) Mechanisms and therapeutic potential of vanilloids (capsaicin-like molecules). *Adv. Pharmacol.,* 24, 123-155.
2. Jansco, G. (1968) Desensitisation with capsaicin and related acylamides as a tool for studying the function of pain receptors. In: K. Lin, D. Armstrong and E.G. Pardo (Eds), Pharmacology of Pain, Pergamon Press, Oxford, 33-55.
3. Szolcsanyi, J. (1993) In *Capsaicin in the study of pain.* Ed. J. Wood, J. London: Academic Press, 1-26.
4. Szallasi, A. and Blumberg, P.M. (1996) Vanilloid receptors: new insights enhance potential as a therapeutic target. Pain, 68, 195-208.
5. Jansco, G., Kiraly, E. and Jansco-Gabor, A. (1977) Pharamacologically induced selective degeneration of chemosensitive primary sensory neurons. *Nature,* 270, 741-743.
6. Oh, U., Hwang, S.W. and Kim, D. (1996) Capsaicin activates a nonselective cation channel in cultured neonatal rat dorsal root ganglion neurons. J. *Neurosciences,* 16, 1659-1667.
7. Wood, J.N et al. (1988) Capsaicin-induced ion fluxes in dorsal root ganglion cells in culture. *J*. *Neurosciences,* 8, 3208-3220.
8. Szolcsanyi, J. and Jansco-Gabor, A. (1975) Sensory effects of capsaicin congeners I. Relationship between chemical structure and pain-producing potency of pungent agents. *Drug Res.,* 25, 1877-1881.
9. Szolcsanyi, J. and Jansco-Gabor, A. (1976) Sensory effects of capsaicin congeners II. Importance of chemical structure and pungency in desensitising activity of capsaicin-like compounds. Drug res., 26, 33-37.
10.James, I.F., Nikina, N. and Wood, J.N. (1993) The capsaicin receptor. In *Capsaicin in the Study of Pain.* Ed. Wood, J. London: Academic Press, 83-104.
11. Szallasi, A. and Blumberg, P.M. (1990) Specific binding of resiniferatoxin, an ultrapotent capsaicin analog, by dorsal root ganglion membranes. *Brain Research,* 524, 106-111.
12. Bevan, S., Hothi, S., Hughes, G., James, I.F., Rang, H.P., Shah, K., Walpole, C.S.J. and Yeats, J.C. (1992) Capsazepine: a competitive antagonist of the sensory neurone excitant capsaicin. *British Journal of Pharmacology,* 101, 423-431.
13.Szallasi, A., Blumberg, P.M., Nilsson, S., Hokfelt, T. and Lundberg, J.M. (1994) Vizualisation by [³H] resiniferatoxin autoradiography of capsaicin-sensitive neurons in the rat, pig, and man. *European Journal of Pharmacology,* 264, 217-221.
14. Szallasi, A., Nillson, S., Farkas-Szallasi, T., Blumberg, J.M., Hokfelt, T. and Lundberg, J.M. (1995) Vanilloid (capsaicin) receptors in the rat: distribution in the brain, regional differences in the spinal cord, axonal transport to the periphery, and depletion by systemic vanilloid treatment. *Brain Research,* 703, 175-183.
15.Caterina, M.J., et al. (1997) The capsaicin receptor: a heat-activated ion channel in the pain pathway. *Nature,* 389, 816-824
16.Bevan, S. and Gepetti P. (1994) Protons: small stimulants of capsaicin-sensitive sensory nerves. *Trends in Neurociences,* 17, 509-512.
17.Petersen, M. and LaMotte, R.H. (1993) Effect of protons on the inward current evoked by capsaicin in isolated dorsal root ganglion cells. *Pain,* 54, 37-42.
18.Kress, M. Fetzer, S., Reeh, P.W. and Vyklicky, L. (1996) Low pH facilitates capsaicin responses in isolated sensory neurons of the rat. *Neurosciences Letters,* 211, 5-8.
19.Wilcox, A.S., Khan, A.S., Hopkins, J.A., and Sikela, J.M. (1991). Use of 3' untranslated sequences of human cDNAs for rapid chromosome assignment and conversion to STSs: Implications for an expression map of the genome. *Nucleic Acids Res.,* 19, 1837-1843.
20.Altschul, S.F., Warren, G., Webb, M., Myers, E.W., and Lipman, D.J., (1990). Basic local alignment search tool. *J*. *Mol. Biol.,* 215, 403-410.
21. Boguski, M.S., Lowe, T.M. and Tolstohev, C.M. (1993) dbEST: database for 'Expressed Sequence Tags' Nature Genetics, 4, 332-333.
22. Gill, R.W., Hodgman, C.T., Littler, C.B., Oxer, M.D., Montgomery, D.S., Taylor, S. and Sanseau, P. (1997). A new dynamic tool to perform assembly of Expressed Sequence Tags (ESTs). *Computer Applications in Biosciences (CABIOS),* 13, 453-457.
23. Lau, P.P. and Gray H.B. (1979). *Nucleic Acids Research,* 6, 331.
24. Trower MK., Burt D., Purvis IJ., Dykes CW. & Christodoulou C. (1995). Fluorescent dye-primer cycle sequencing using non-purified PCR products as templates; development of a protocol amenable to high-throughput DNA sequencing. *Nucleic Acids Research* ,23, 2348-2349
25. Shepherd, N., Pfogner, B., Coulby, J., Ackerman, S., Vaidyanathan, G., Sauer, R., Balkenhol, T. and Sternberg, N. (1994). Preparation and screening of an arrayed human genomic library generated with the P1 cloning system. Proc. *Natl. Acad. Sci. USA.,* 91, 2629-2633.
26. Birboim, H.C. and Doly, J. (1979). *Nucl. Acids Res.,* 7, 1513-1523.
27. Burge, C. and Karlin, S. (1997). Prediction of complete gene structures in human genomic DNA. *J*. *Mol. Biol.* 268, 78-94.
28. Sambrook, J., Fritsch, E.F. and Maniatis, T. Molecular Cloning: a Laboratory Manual. 2^{nd} Edition. CSH Laboratory Press. (1989)
29. Davis L. G., Battey J. F. & Dibner M.D., Basic Methods in Molecular Biology. 1^{st} Edition. Elsevier (1986).
30. Caterina, M. C., Rosen, T. A., Tominaga, M., Brake, A. J. & Julius, D. (1999) A capsaicin-receptor homologue with a high threshold for noxious heat. *Nature,* 398,436-441.
31. Hamill, O.P. Marty, A., Neher, E., Sakmann, B., & Sigworth, F.J. (1981). Improved patch-clamp techniques for high resolution current recording from cells and cell-free membrane patches. *Pflügers Arch.,* 391, 85-100.
32. Rees. S., Coote, J., Stables, J., Goodson, S., Harris, S. & Lee, M. G. (1996) Bicistronic vector for the creation of stable mammalian cell lines that predisposes all antibiotic-resistant cells to express recombinant protein. Biotechniques, 20, 102-110.

## Claims

1. A method of identifying an antibody which specifically binds to and modulates the activity of a human vanilloid receptor which method comprises;
(a) Providing a stable cell line comprising an expression vector which vector comprises the polynucleotide of figure 17 under conditions suitable for expression of said human vanilloid receptor;
(b) Providing a candidate antibody;
(c) Determining whether the antibody of step (b) specifically binds to and modulates the receptor of step (a).

2. The method of claim 1 wherein the antibody is monoclonal.

3. The method of claim 1 or 2 wherein the cell line is selected from the group consisting of; HEK293, CHO, COS, HeLa or BHK.

4. An antibody identified by the method of claim 1.

5. A pharmaceutical composition comprising the antibody of claim 5.
